# EUROPÄISCHE PATENTSCHRIFT

(11) **EP 1 680 678 B1**
(45) Veröffentlichungstag und Bekanntmachung des Hinweises auf die Patenterteilung: **07.10.2009**
(21) Anmeldenummer: 04790869.4
(22) Anmeldetag: 26.10.2004
(51) Int. Cl.: G01N 33/53

(54) **REKOMBINANTES ZELLSYSTEM ZUR DEORPHANISIERUNG VON G PROTEIN-GEKOPPELTEN REZEPTOREN**
RECOMBINED CELL SYSTEM FOR DEORPHANIZING G PROTEIN-COUPLED RECEPTORS
SYSTEME CELLULAIRE RECOMBINANT POUR LA DEORPHANISATION DE RECEPTEURS COUPLES A UNE PROTEINE G

(30) Priorität: 27.10.2003 DE 10350054; 20.04.2004 DE 102004019028
(43) Veröffentlichungstag der Anmeldung: 19.07.2006
(73) Patentinhaber: DEUTSCHES INSTITUT FÜR ERNÄHRUNGSFORSCHUNG - STIFTUNG DES ÖFFENTLICHEN RECHTS - VERTRETEN DURCH DEN STIFTUNGSVORSTAND, 14558 Bergholz-Rehbrücke (DE)
(72) Erfinder: KRAUTWURST, Dietmar, 14558 Nuthetal (DE); SHIROKOVA, Elena, Montréal, Qc. H2T 2V3 (CA); SCHMIEDEBERG, Kristin, 14467 Potsdam (DE); WILLECKE, Klaus, 53639 Königswinter (DE); NIESSEN, Heiner, 50999 Köln (DE); BEDNER, Peter, 51067 Köln (DE); RAGUSE, Jan-Dirk, 12159 Berlin (DE); MEYERHOF, Wolfgang, 22848 Norderstedt (DE)
(74) Vertreter: Krauss, Jan
(86) Internationale Anmeldenummer: PCT/EP2004/012086
(87) Internationale Veröffentlichungsnummer: WO 2005/043158

(56) Entgegenhaltungen:
- WO-A2-03/004611
- ILLING NICOLA ET AL: "Conditionally immortalized clonal cell lines from the mouse olfactory placode differentiate into olfactory receptor neurons" MOLECULAR AND CELLULAR NEUROSCIENCE, Bd. 20, Nr. 2, Juni 2002 (2002-06), Seiten 225-243, XP002315703 ISSN: 1044-7431
- SHIROKOVA E. ET AL.: "Identification of specific ligands for orphan olfactory receptors: G-protein dependent agonism and antagonism of odorants" JOURNAL OF BIOLOGICAL CHEMISTRY, [Online] 14. Dezember 2004 (2004-12-14), XP002315704 Gefunden im Internet: URL:http://www.jbc.org/cgi/reprint/M411508 200v1> [gefunden am 2005-01-26]
- KAJIYA K ET AL: "Molecular bases of odor discrimination: Reconstitution of olfactory receptors that recognize overlapping sets of odorants." THE JOURNAL OF NEUROSCIENCE : THE OFFICIAL JOURNAL OF THE SOCIETY FOR NEUROSCIENCE. 15 AUG 2001, Bd. 21, Nr. 16, 15. August 2001 (2001-08-15), Seiten 6018-6025, XP002315705 ISSN: 1529-2401 in der Anmeldung erwähnt
- WILSON S ET AL: "ORPHAN G-PROTEIN-COUPLED RECEPTORS: THE NEXT GENERATION OF DRUG TARGETS?" BRITISH JOURNAL OF PHARMACOLOGY, BASINGSTOKE, HANTS, GB, Bd. 125, Nr. 7, Dezember 1998 (1998-12), Seiten 1387-1392, XP001010584 ISSN: 0007-1188 in der Anmeldung erwähnt
- REED R R: "Signaling pathways in odorant detection." NEURON. FEB 1992, Bd. 8, Nr. 2, Februar 1992 (1992-02), Seiten 205-209, XP002315707 ISSN: 0896-6273
- TOUHARA KAZUSHIGE: "Odor discrimination by G protein-coupled olfactory receptors." MICROSCOPY RESEARCH AND TECHNIQUE. 1 AUG 2002, Bd. 58, Nr. 3, 1. August 2002 (2002-08-01), Seiten 135-141, XP002315708 ISSN: 1059-910X
- BRUZZONE R ET AL: "CONNECTIONS WITH CONNEXINS: THE MOLECULAR BASIS OF DIRECT INTERCELLULAR SIGNALING" EUROPEAN JOURNAL OF BIOCHEMISTRY, BERLIN, DE, Bd. 238, 1996, Seiten 1-27, XP002913288 ISSN: 0014-2956

## Beschreibung

Die vorliegende Erfindung betrifft ein rekombinantes Zellsystem, umfassend eine tierische Wirtszelle, umfassend einen rekombinanten G Protein-gekoppelten spezifischen Rezeptor, den rekombinanten Ca2+ spezifischen Kanal CNGA2 und ein rekombinantes Protein aus der Gruppe der Connexine. Die Erfindung betrifft weiterhin ein Verfahren zur Herstellung des erfindungsgemäßen Zellsystems und die Verwendung des Systems zur Deorphansierung G Protein-gekoppelter Rezeptoren. Weiterhin betrifft die vorliegende Erfindung die Verwendung des Zellsystems zur Auffindung neuer G Protein-gekoppelter Rezeptoren aus Genbanken.

### Stand der Technik

Die Superfamilie von G Protein-gekoppelten Rezeptoren (GPCRs; 7TMs) ist eine der größten Familien von den Menschen identifizierten Genen und weist nachweislich eine Geschichte als eine exzellente Quelle von Wirkstoffzielen auf. Sie reagieren auf eine große Zahl von Reizen, einschließlich kleinen Peptiden, Lipidanaloga, Aminosäure-Derivaten und sensorischen Reizen, wie z. B. Licht, Geschmack und Geruch und übertragen Signale in das Innere der Zelle durch Interaktion (u.a.) heterotrimeren G Proteinen. Die fast vollständige Sequenzierung des menschlichen Genoms erlaubte die Identifizierung einer großen Zahl von Sequenzen, die so genannte "orphan" GPCRs kodieren, potentielle Rezeptoren, deren natürliche Liganden noch aufgefunden werden müssen. In vielen Fällen ist das Ausmaß der Sequenzhomologie mit bekannten Rezeptoren nicht ausreichend, um den natürlichen Liganden für diese Orphan-Rezeptoren zu finden, obwohl es gewöhnlicherweise möglich ist, die mögliche Natur des jeweiligen Liganden zu bestimmen, zum Beispiel Peptid, Lipid, Nukleotid usw. Die so genannte "Deorphanisierung" dieser neuen GPCRs und die Bestimmung ihrer biologischen Funktionen hat sich zu einem hauptsächlichen Ziel vieler der großen pharmazeutischen Firmen entwickelt, sowie verschiedener akademischer Gruppen. Seit 1995 wurden mehr als 50 Liganden für Orphan GPCRs durch die Verwendung des Orphan Rezeptors als ein Biosensor und Screenen von Kandidaten-Verbindungen entdeckt, wobei auf eine biologische Antwort geschaut wurde (der so genannte "reverse pharmacology" Ansatz) (Szekeres PG. Functional assays für identifying ligands at orphan G protein-coupled receptors. Receptors Channels. 2002;8(5-6):297-308.).

Kurz gesagt schließt die reverse molekulare pharmakologische Technik das Klonieren und die Expression von orphan GPCRs in Säugerzellen und Screenen in diesen Zellen auf eine funktionelle Antwort gegenüber kognaten oder Surrogatagonisten, die in biologischen Extraktpreparationen, Peptid-Bibliotheken und komplexen Verbindungssammlungen vorhanden sind ein. Der funktionelle Genomics Ansatz schließt die Verwendung von "humanisierten" Hefezellen ein, wobei das Hefezellen GPCR Transduktionssystem so verändert ist, um eine funktionelle Expression und ein Koppeln von menschlichen GPCRs an die endogene Signalmaschinerie zu ermöglichen. Beide Systeme stellen eine exzellente Plattform zu Identifizierung von neuen Rezeptorliganden zur Verfügung. Sobald aktivierende Liganden identifiziert sind, können sie als pharmakologische Werkzeuge verwendet werden, um die Rezeptorfunktion und die Beziehung zu Erkrankungen, einschließlich Fettsucht, Entzündungserkrankungen, Herzerkrankungen und Krebs, zu untersuchen. (Wilson S, Bergsma DJ, Chambers JK, Muir AI, Fantom KG, Ellis C, Murdock PR, Herrity NC, Stadel JM. Orphan G-protein-coupled receptors: the next generation of drug targets? Br J Pharmacol. 1998 Dec;125(7):1387-92.; Shaaban S, Benton B. Orphan G protein-coupled receptors: from DNA to drug targets. Curr Opin Drug Discov Devel. 2001 Sep;4(5):535-47).

Ein weiterer Ansatz zur Auffindung von Rezeptorliganden ist der Vergleich von in der Datenbank erhältlichen bekannten und/oder putativen GPCRs (Joost P, Methner A. Phylogenetic analysis of 277 human G-protein-coupled receptors as a tool für the prediction of orphan receptor ligands. Genome Biol. 2002 Oct 17;3(11):RESEARCH0063.). Dieser Vergleich kann auch zwischen verschiedenen Spezies erfolgen, um so Rezeptoren zu identifizieren, die z. B. in der Maus identifiziert worden (Vassilatis DK, Hohmann JG, Zeng H, Li F, Ranchalis JE, Mortrud MT, Brown A, Rodriguez SS, Weller JR, Wright AC, Bergmann JE, Gaitanaris GA. The G protein-coupled receptor repertoires of human und mouse. Proc Natl Acad Sci USA. 2003 Apr 15;100(8):4903-8. Epub 2003 Apr 04.).

Eine neben den Gruppen der partikulären Guanylyl-Cyklasen (zusammenfassend beschrieben in, z.B., Lucas KA, et al. Guanylyl cyclases und signaling by cyclic GMP. Pharmacol Rev. 2000 Sep;52(3):375-414, Gibson AD, Garbers DL. Guanylyl cyclases as a family of putative odorant receptors. Annu Rev Neurosci. 2000;23:417-39.), Pheromonrezeptoren, z.B. des V1R-Typs (zusammenfassend beschrieben in, z.B., Matsunami H, Amrein H. Taste und pheromone perception in mammals und flies. Genome Biol. 2003;4(7):220. Epub 2003 Jun 30; Dulac C, Torello AT. Molecular detection of pheromone signals in mammals: from genes to behaviour. Nat Rev Neurosci. 2003 Jul;4(7):551-62.) und der Adrenalinrezeptoren, z.B. der alpha- oder beta-adrenergen Rezeptoren (zusammenfassend beschrieben in, z.B., Koshimizu TA, et al. Recent progress in alpha 1-adrenoceptor pharmacology. Biol Pharm Bull. 2002 Apr;25(4):401-8.), die sich durch verschiedene intrazelluläre Signalkaskaden unterscheiden, besonders interessante Gruppe ist die der olfaktorischen Rezeptoren, zu denen auch bereits eine große Zahl von Patentanmeldungen hinterlegt wurden (z.B. EP 1301599; CN1386760; CN1380323; CN1376713; CN1376691; AU2147402; EP1235859 und WO 02/059274).

Das olfaktorische System ermöglicht es den Wirbeltieren, eine große Zahl chemisch unterschiedlicher Geruchsmoleküle nachzuweisen und voneinander zu unterscheiden. Im Menschen und der Maus kodieren jeweils -350 zu 1000 odorante Rezeptoren (OR) Gene für sieben Transmembran-durchspannende (7TM) und G Protein-gekoppelte Rezeptoren (GPCR) (Buck und Axel, 1991; Zozulya et al., 2001; Zhang und Firestein, 2002), die in den olfaktorischen sensorischen Neuronen (OSN) des olfaktorischen Epithels (OE) (für eine Übersicht, siehe Mombaerts, 1999; Young und Trask, 2002) exprimiert werden. Ein bestimmtes OSN exprimiert höchstwahrscheinlich nur einen einzelnen Typ von OR (Chess et al., 1994; Malnic et al., 1999), und einzelne OSN zeigen oft eine breit eingestellte Odoranten Spezifität, die teilweise von der Odorant-Konzentration (Sicard und Holley, 1984; Sato et al., 1994; Malnic et al., 1999; Duchamp-Viret et al., 2000; Ma und Shepherd, 2000; Hamana et al., 2003) abhängig ist. Daher wird die Odorant-Unterscheidungs-, Qualitäts- und Intensitätskodierung eines jeden bestimmten OSN von dem EC50 Odorantprofil des bestimmten OR Typs, die sie exprimiert, abhängen (Malnic et al., 1999; Kajiya et al., 2001; Hamana et al., 2003). Mehr als vier spatial distinkte Expressionsgebiete der OR Gen wurden in dem OE von Mäusen beschrieben (für eine Übersicht, siehe Touhara, 2002). Eine systematische Verteilung der Odorantsensitivität über das OE hinweg wurde durch Elektroolfaktogramm (EOG) Aufzeichnungen und *in situ* Ca2+ Bildgebung (Scott und Brierley, 1999; Omura et al., 2003) gezeigt. Jedoch fehlt bis heute Information über die molekularen Determinanten, die jeder spatial organisierten Odorant-Antwortzone zugrunde liegen, z.B. Odorant-Erkennungsprofile von OR und ihren zonalen Expressionsmustern innerhalb des OE.

Nach der Odorantstimulierung aktiviert der OR eine olfaktorisch-spezifische Signaltransduktionskaskade (Reed, 1992; Gold, 1999), die das G-Protein G-alpha-olf (Jones und Reed, 1989; Firestein et al., 1991), die Adenylatcyklase (AC) Typ III (Bakalyar und Reed, 1990) und einen heteromerischen cyklicsches Nukleotid-öffnenden (CNG) Ca2+-permeablen Kationenkanal (Nakamura und Gold, 1987; Dhallan et al., 1990; Dzeija et al., 1999) einschließt. Konsistenterweise, machten Gene-target Deletionen von G-alpha-olf, ACIII, oder der CNGA2 Kanaluntereinheit (Brunet et al., 1996; Belluscio et al., 1998; Baker et al., 1999; Wong et al., 2000) Mäuse größtenteils anosmisch. Ein andere olfaktorischer Signaltransduktions-Signalweg, der spezifisch durch cGMP moduliert wird, schließt die partikuläre Guanylylcyklase Typ D (GC-D) ein, die in einer Untergruppe von OSN vorhanden ist (Juilfs et al., 1997; Meyer et al., 2000). Trotz des genauen Wissens über die Genetik von OR und ihren Signaltransduktionskomponenten, haben nur ein paar Untersuchungen Odoranten mit einzelnen OR in Zusammenhang gebracht, mittels Adenoviraler Überexpressionsassays in vivo (Zhao et al., 1998; Araneda et al., 2000), heterologer Expression von rekombinanten OR in vitro (Krautwurst et al., 1998; Wetzel et al., 1999; Gaillard et al., 2002), Einzelzell RT-PCR von Odorant-responsiven OSN (Malnic et al., 1999; Touhara et al., 1999; Kajiya et al., 2001), und OR Gen-targeting, gefolgt durch funktionelle Analyse von Einzel-OSN (Bozza et al., 2002). Die funktionelle Expression einiger N-terminal-markierter OR in HEK-293 Zellen wurde durch künstliches Koppeln dieser an Phosphoinositol-Signalgebung und Kalziumfreisetzung via den G-Protein Untereinheiten alpha15,16 erreicht (Krautwurst et al., 1998; Touhara et al., 1999; Kajiya et al., 2001; Gaillard et al., 2002) oder via G-alpha-q/11 (Wetzel et al., 1999). Jedoch ist nicht bekannt, ob alle OR, und mit welcher Effizienz, über die G-Proteine alpha15,16 koppeln können. Kürzliche Überprüfungen von Ergebnissen anderer Gruppen haben gezeigt, daß das alpha15,16 System zu einigen falsch-negativen Ergebnissen führte.

Die bei der Untersuchung der Rezeptoren des olfaktorischen Systems aufgefundenen Probleme lassen sich auch auf die anderen oben genannten Klassen von Rezeptoren übertragen. Es fehlt daher bis zum heutigen Tage ein Zellsystem, um GPCR in einem geeigneten genetischen Hintergrund und insbesondere OR in ihrem original G-alpha-olf-ACIII-cAMP SignalHintergrund funktionell zu screenen und zu charakterisieren. Gleichzeitig fehlt ein zelluläres System mit einem geeigneten genetischen Hintergrund, um mittels diesem neue Orphan-Rezeptoren schnell und in hohem Maßstab aufzufinden und zu charakterisieren.

Es ist eine Aufgabe der vorliegenden Erfindung, ein Zellsystem zur Verfügung zu stellen, um das Methodenspektrum bei der Auffindung und Charakterisierung (Deorphanisierung) von Rezeptoren und ihren dazugehörigen Liganden zu erweitern und zu verbessern. Das System sollte optimaler weise billig herzustellen sein, größtenteils etablierte Meßtechniken ermöglichen und zudem eine geringe genetische Sicherheitsstufe aufweisen. Zudem sollten falsche Ergebnisse so weit wie möglich ausgeschlossen werden.

Gemäß eines ersten Aspekts der vorliegenden Erfindung wird diese Aufgabe der vorliegenden Erfindung wird durch ein rekombinantes Zellsystem gelöst, wobei das System eine tierische Wirtszelle umfaßt, die die folgenden rekombinanten Proteine umfaßt A) einen rekombinanten spezifischen G Protein-gekoppelten Rezeptor, B) den rekombinanten Ca2+ spermeablen Kanal CNGA2, und C) ein rekombinantes Protein aus der Gruppe der Connexine, z.B. Cx43 oder Cx26. Weitere bevorzugte Ausführungsformen des erfindungsgemäßen Systems sind in den abhängigen Ansprüchen beansprucht. Erfindungsgemäß kann der rekombinante Ca2+ spezifische Kanal CNGA2 als Homomer oder Heteromer zusammengesetzt sein. Bevorzugt ist ein Untereinheiten-Homomer.

Die vorliegende Erfindung beruht zum Teil auf der Erkenntnis, daß in einem Fall die heterologe Expression eines OR mit Odorant-induzierter cAMP Produktion assoziiert war (Kajiya et al., 2001), was vermuten ließ, daß ein rekombinanter OR an endogene G-alpha-s und AC Proteine in einer humanen Zelline koppelt.

WO 03/004611 beschreibt die Expression der funktionellen menschlichen olfaktorischen Nukleotid-öffnenden (CNG) Kanal Untereinheit OCNC1 in rekombinanten Wirtszellen und deren Verwendung in Zell-basierten Tests, um Geruchsmodulatoren zu identifizieren. Es wird mit dem Kanal selber getestet. Illing, N. et al., Molecular and Cellular Neuroscience, 2002, ISSN 1044-7431, 20(2), 225-243, beschreibt ein rekombinantes Zellsystem umfassend OCNC1, mOREG (einen G-Protein gekoppelten Rezeptor), endogenes Gs und eine Adenylat Cyclase. Die US 6,492,143 beschreibt olfaktorische Rezeptor Expressionsbibliotheken und Verfahren zu deren Herstellung und Verwendung. WO 01/51609 beschreibt dann die Isolierung und in vitro Differenzierung von konditional immortalisierten Maus-olfaktorischen Rezeptomeuronen. Durch keine der oben genannten Publikationen wird somit ein erfindungsgemäßes Zellsystem offenbart oder nahegelegt.

Durch das Einbringen des Connexins wird die Sensitivität des erfindungsgemäßen Systems verbessert, da Ca2+ zwischen den Zellen verteilt werden kann.

Ein besonders bevorzugtes erfindungsgemäßes rekombinantes Zellsystem umfaßt einen rekombinanten spezifischen G Protein-gekoppelten Rezeptor, . Die Komponenten des erfindungsgemäßen rekombinanten Zellsystems bestehen in diesem Fall somit aus A) einen rekombinanten spezifischen G Protein-gekoppelten Rezeptor aus der Gruppe der partikulären Guanylyl-Cyklasen, B) dem rekombinanten Ca2+ spezifischen Kanal CNGA2, und einem rekombinanten Protein aus der Gruppe der Connexine, z.B. Cx43 oder Cx26.

Ein weiterer Aspekt der vorliegenden Erfindung betrifft dann ein erfindungsgemäßes rekombinantes Zellsystem, das weiter eine auf den spezifischen G Protein-gekoppelten Rezeptor abgestimmte Cyklase, z.B. eine Adenylyl- oder Guanylyl-Cyklase umfaßt. Die Komponenten des erfindungsgemäßen rekombinanten Zellsystems bestehen in diesem Fall somit aus A) einen rekombinanten spezifischen G Protein-gekoppelten Rezeptor, B) dem rekombinanten Ca2+ spezifischen Kanal CNGA2, und C) einem rekombinanten Protein aus der Gruppe der Connexine, und D) der auf den spezifischen Rezeptor abgestimmte Cyklase. Bevorzugterweise ist ein erfindungsgemäßes rekombinantes Zellsystem, wobei der rekombinante spezifische G Protein-gekoppelte Rezeptor ausgewählt ist aus der Gruppe der Pheromonrezeptoren, z.B. des V1R-Typs mit allen Familien VR-a bis VR-1, einschließlich der V3R-Typs (VR-d), zum Beispiel V1R-b2, der Hormonrezeptoren, z.B. der beta-adrenergen Rezeptoren und der olfaktorischen Rezeptoren, z.B. OR1A1, OR1A2, Olfr43, Olfr49, MOR261-10, MOR267-1, LOC331758, Olfr41 oder Olfr6.

Im Rahmen der Untersuchungen zu der vorliegenden Erfindung haben die Erfinder weiterhin festgestellt, daß die Sensitivität des Zellsystems erheblich zunimmt, wenn zusätzlich ein auf den spezifischen G Protein-gekoppelten Rezeptor abgestimmtes rekombinantes G-Protein, z.B. G-alpha-olf vorhanden ist. Die Einbringung eines solchen G-Proteins ist somit erfindungsgemäß bevorzugt. Bei Einbringung eines entsprechenden G-Proteins kann in den meisten Fällen bequemer weise auf ein Vorbehandeln (Primen) des Zellsystems verzichtet werden, was die Messung vereinfacht.

Ein weiterer Aspekt der vorliegenden Erfindung betrifft ein rekombinantes Zellsystem, wobei die tierische Wirtszelle ausgewählt ist aus Mauszellinien oder humanen Zellinien, z.B. menschlichen Krebszellinien, wie z.B. HeLa oder HEK293. Es können viele Zellinien verwendet werden, wichtig ist lediglich, daß der entsprechende genetische Hintergrund so gewählt sein muß, daß die entsprechende Signalkaskade vorhanden ist. Der Fachmann wird aus der Beschreibung hier leicht erkennen, welche Zellinien geeignet sind.

Ein besonders bevorzugtes erfindungsgemäßes rekombinantes Zellsystem ist schließlich ausgewählt aus den Zellsystemen
- HeLa-Cx43/CNGA2/O1fr49;
- HeLa-Cx43/CNGA2/G-alpha-olf;
- HeLa-Cx43/CNGA2/G-alpha-olf/Olfr 49;
- HeLa-Cx43/CNGA2/G-alpha-olf/Olfr41;
- HeLa-Cx43/CNGA2/G-alpha-olf/Olfr 6 oder
- HeLa-Cx43/CNGA2/G-alpha-olf/OR1A1.

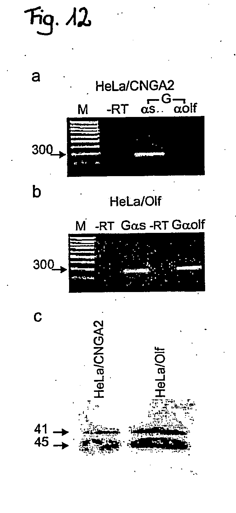

Eine besonders bevorzugte erfindungsgemäße rekombinantes Zellinie "HeLa/olf" mit den Komponenten HeLa - Cx43 (aus der Ratte)/CNGA2 (aus dem Rind)/G-alpha-olf (aus dem Menschen, überexprimiert, siehe Figur 12c) wurde am 20. April 2004 bei der DSMZ - Deutsche Sammlung von Mikroorganismen und Zellkulturen GmbH im Mascheroder Weg 1b, D-38124 Braunschweig hinterlegt. Die Hinterlegung hat die Nummer DSM ACC2649 erhalten.

Ein weiter besonders bevorzugtes erfindungsgemäßes rekombinantes Zellsystem ist **dadurch gekennzeichnet, daß** die rekombinanten Proteine stabil, überexprimiert und/oder transient transfiziert vorliegen. Unter "stabil" soll im Rahmen der vorliegenden Erfindung eine Expression über mindestens 10 bis 13 Passagen hinweg bei 1 bis 2 Passagen pro Woche verstanden werden (dazu siehe auch die entsprechenden Beispiele, unten). Diese stabile Transfektion war bei bisherigen Systemen nicht gegeben und stellt somit einen weiteren Vorteil des erfindungsgemäßen System dar. Unter "überexprimiert" soll im Rahmen der vorliegenden Erfindung eine Verstärkung der Expression von G-Proteinen in der Zelle über das natürliche Ausmaß hinaus bedeuten. Dies umfaßt zum Einen eine additive Zunahme der G-Protein-Expression, als auch eine erhöhte Expression eines (im Fall von HeLa-Olf der beiden) G-Proteins (siehe z.B. dazu Figur 12c). Im Fall der HeLa-Olf-Zellen wird die Überexpression wahrscheinlich durch den verwendeten CMV-Promotor verursacht.

Ein weiterer Aspekt der vorliegenden Erfindung betrifft ein Verfahren zur Auffindung von G Protein-gekoppelten Rezeptor induzierenden Substanzen, umfassend die Schritte von a) zur

Verfügung stellen eines erfindungsgemäßen rekombinanten Zellsystems, b) in Kontakt bringen des Zellsystems mit einer potentiell G Protein-gekoppelten Rezeptor induzierenden Substanz, und c) Messen der Aktivierung oder Inhibierung des Ca2+ Einstroms in die Zelle. Solche Screeningverfahren können auf Basis der hier beschriebenen Verfahren und der reichhaltigen Literatur im Bereich des Screening (z.B. Szekeres PG. Functional assays for identifying ligands at orphan G protein-coupled receptors. Receptors Channels. 2002;8(5-6):297-308) durch den Fachmann leicht entworfen werden. Besonders bevorzugt ist die Durchführung im Hochdurchsatz-Verfahren. Weiter bevorzugt ist das Screening mit Substanzen, die als Odorantien bereits bekannt sind, wie zum Beispiel (-)Citronellal oder beta-Citronellol, Pheromonen, Hormone, wie zum Beispiel Adrenalin oder natriuretisches Peptid Typ-C.

Das erfindungsgemäß verwendete Meßverfahren des Messens der Aktivierung oder Inhibierung des Ca2+ Einstroms in die Zelle kann jedes geeignete Verfahren sein, bevorzugt ist jedoch ein erfindungsgemäßes Verfahren, das eine Beladung der Zelle mit Fura-2-AM oder Fluo-4-AM und ein Messen der Emissions-Wellenlänge bei 515 nm einschließt. In manchen Fällen kann es sinnvoll sein, daß das Zellsystem für die Messung mit einem Enhancer, wie zum Beispiel Forskolin oder Thapsigargin vorbehandelt wird.

Wie oben erwähnt, können neu aufgefundene G Protein-gekoppelten Rezeptor induzierende Substanzen die Grundlage für wertvolle pharmazeutische Erzeugnisse oder "lead-structures" für die Entwicklung solcher pharmazeutischen Erzeugnisse darstellen. In einem weiteren Aspekt betrifft die vorliegende Erfindung somit ein Verfahren zur Herstellung einer pharmazeutischen Zusammensetzung, umfassend die Schritte von a) Durchführen eines Verfahrens Verfahren zur Auffindung von Rezeptor aktivierenden Substanzen gemäß der vorliegenden Erfindung, und b) Formulieren der erhaltenen G Protein-gekoppelten Rezeptor induzierenden Substanz mit bekannten Hilfs- und Zusatzstoffen. Die eigentliche Formulierung ist für den Fachmann kein Problem, hängt von den jeweiligen zu Formulierenden Substanzen ab und kann leicht aus der einschlägigen Literatur entnommen werden.

Ein weiterer Aspekt der vorliegenden Erfindung betrifft ein erfindungsgemäßes rekombinantes Zellsystem, wobei das Zellsystem einen potentiellen rekombinanten spezifischen G Protein-gekoppelten Rezeptor anstelle eines bereits bekannten oder Orphan-Rezeptor umfaßt. So kann das erfindungsgemäße Zellsystem als Grundlage für die Auffindung von weiteren orphan G Protein gekoppelten Rezeptoren dienen, indem exprimierte Gene als Kassette in das Zellsystem eingeführt werden und deren Fähigkeit untersucht wird, auf bestimmte Reize (z.B. Odoranten) eine Aktivierung oder Inhibierung des Ca2+ Influx hervorzurufen. Geeignete Quellen solcher exprimierten Gene sind käuflich erhältliche Tier und/oder Gewebespezifische Banken, die z.B. das Proteom einer Zelle umfassen können. Auch hier ist bevorzugt, daß das Verfahren in einer Hochdurchsatz-Umgebung erfolgt, z.B. in Mikrotiterplatten in einem Fluoreszenz-Plattenlesegerät oder hochauflösend mikroskopgestützt auf Einzelzellebene.

Die Erfindung betrifft somit weiterhin ein Verfahren zur Auffindung von neuen G Protein gekoppelten Rezeptoren umfassend die Schritte von a) zur Verfügung stellen eines geeigneten erfindungsgemäßen rekombinanten Zellsystems wie oben, b) in Kontakt bringen des Zellsystems mit einer G Protein-gekoppelten Rezeptor aktivierenden Substanz oder vermutlich G Protein-gekoppelten Rezeptor aktivierenden Substanz, und c) Messen der Aktivierung oder Inhibierung des Ca2+ Einstroms in die Zelle.

Ein weiterer Aspekt der vorliegenden Erfindung betrifft dann ein Verfahren zur Herstellung eines rekombinanten Zellsystems, umfassend a) zur Verfügung stellen einer geeigneten tierischen Wirtszelle, b) Einbringen eines rekombinanten spezifischen G Protein-gekoppelten Rezeptors oder eines potentiellen rekombinanten spezifischen Rezeptors, c) Einbringen des rekombinanten Ca2+ permeablen Kanals CNGA2, und d) Einbringen eines rekombinanten Proteins aus der Gruppe der Connexine, z.B. Cx43 oder Cx26. Erfindungsgemäß kann der rekombinante Ca2+ permeable Kanal CNGA2 als Homomer oder Heteromer eingebracht werden. Bevorzugt ist ein Untereinheiten-Homomer.

Durch das Einbringen des Connexins wird die Sensitivität des erfindungsgemäßen Systems verbessert, da Ca2+ zwischen den Zellen verteilt werden kann. Bevorzugt ist ein erfindungsgemäßes Verfahren, das ein Einbringen einer auf den spezifischen G Protein-gekoppelten Rezeptor abgestimmte Cyklase, z.B. eine Adenylyl- oder Guanylyl-Cyklase und/oder das ein Einbringen eines auf den spezifischen G Protein-gekoppelten Rezeptor abgestimmten rekombinanten G-Proteins, z.B. G-alpha-olf umfaßt.

Erfindungsgemäß kann jede dem Fachmann bekannte Technik zu der Einführung der genetischen Konstrukte verwendet werden. Erfindungsgemäß bevorzugt ist ein Verfahren, wobei das Einbringen ausgewählt ist aus Transfektion, z.B. Ca2+-Phosphat-Transfektion, Lipofektion und Transduktion, sowie anschließender optionaler Integration ins Genom mit Hilfe einer Rekombinase und/oder Antibiotika-Selektionsklonierung, und Transduktion.

Wie oben bereits erwähnt, kann das erfindungsgemäße rekombinante Zellsystems zur Deorphanisierung von G Protein-gekoppelten Rezeptoren durch Auffindung von entsprechenden G Protein-gekoppelten Rezeptor induzierenden Substanzen, z.B. Odorantien, verwendet werden. Gleichzeitig kann das erfindungsgemäße Zellsystem auch zur Auffindung von neuen zellulären G Protein-gekoppelten Orphan-Rezeptoren verwendet werden. Diese Rezeptoren können sogar in einem Durchgang identifiziert und deorphanisiert werden, falls die zum Screening verwendete Substanz gleichzeitig als die für den Rezeptor spezifische Substanz identifiziert wird.

Die Erfinder haben nun die olfaktorische Signaltransduktion in HeLa/Olf Zellen von den olfaktorischen Rezeptoren via das G-Protein alpha-olf und die Adenylylcyklasen Typ III zu dem homomeren olfaktorischen cyklischen Nukleotide-öffnenden CNGA2 Kanal stabil rekonstituiert. Die Signaleffizienz der olfaktorischen Rezeptoren in HeLa/Olf Zellen wurde durch die Anwesenheit von G-alpha-olf erhöht, verglichen mit ihrem Signalisieren via endogenem G-alpha-s. Der CNGA2 Kanal dient als ein Sensor, der Veränderungen in der intrazellulären cyklischen Nukleotid-Konzentration durch einen Calcium-Influx anzeigt, der mit Fluoreszenz bildgebenden Techniken verfolgt werden kann.

Rekonstitution von olfaktorischer Rezeptor-cAMP-Signalgebung in HeLa Zellen. - Um olfaktorische Rezeptoren durch einen funktionelle Genomics Ansatz zu de-orphanisieren, transfizierten die Erfinder ein diverses olfaktorisches Rezeptorsubgenom in HeLa/Olf Zellen. Die Erfinder identifizierten neue 3 von 93 (rot, gelb, grün; Kontrolle: violett) Maus Rhodopsinmarkierte olfaktorische Rezeptor Chimären, die in einem 96-Well Screening Ansatz unterschiedlich auf 1 µM (-)Citronellal oder 10 µM beta-Citronellol reagierten.

Funktionelle Genomics von OR wurden jedoch größtenteils durch das Fehlen eines Zellsystems behindert, um rekombinante OR in ihrem olfaktorische Signalhintergrund zu untersuchen. Die Erfinder haben hier zum ersten mal die funktionelle Rekonstitution eines partiellen OR Genoms beschrieben, zusammen mit seinen olfaktorischen Signaltransduktionsmolekülen in einer menschlichen Zelllinie, um die Odorantienkodierung auf dem Rezeptorniveau zu untersuchen.

Cyklische Nukleotide-Sensing und Ca2+ Signalgebung via CNGA2 - Die hier mit HeLa-Cx43/CNGA2 Zellen gezeigten Experimente demonstrieren deren Brauchbarkeit für das funktionelle Screening, die Deorphanisierung und Charakterisierung von nicht-olfaktorischen GPCR oder partikulären GC, die in der cAMP oder cGMP Signalgebung beteiligt sind. Die EC50 Werte, die die Erfinder in HeLa-Cx43/CNGA2 Zellen für Isoproterenol, das auf endogenen P-AR wirkt, und CNP, das auf rekombinanten GC-B wirkt, bestimmten, stehen in Übereinstimmung mit der Literatur (Lucas et al., 2000; Crider und Sharif, 2002). Unsere Bestimmungen der cAMP Produktion in diesen Zellen diente dazu, die Ligand-abhängige Stimulation von Rezeptor/G Protein Kombinationen mit der Funktion des CNGA2 Kanals als einem cyklisches Nukleotid-abhängigen Ca2+ Influxreporter zu verbinden. Die Erfinder haben eine starke Korrelation zwischen der Anwendung des Agonisten der Stimulation von Ca2+ Influx gezeigt, durch zeigen, daß Liganden bei ihrer EC50 eine intrazelluläre cAMP Konzentration erzeugen, die nahe an ihrer EC50 für den olfaktorischen homomeren CNGA2 Kanal liegt.

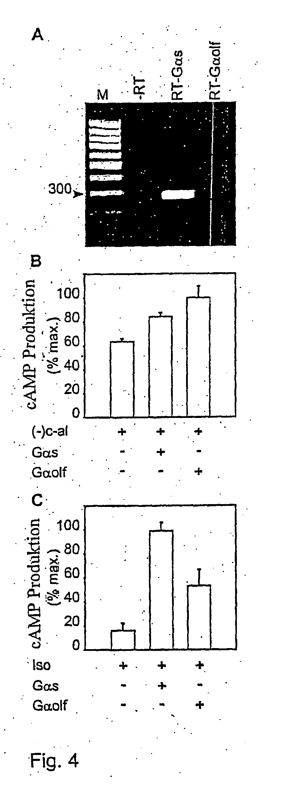

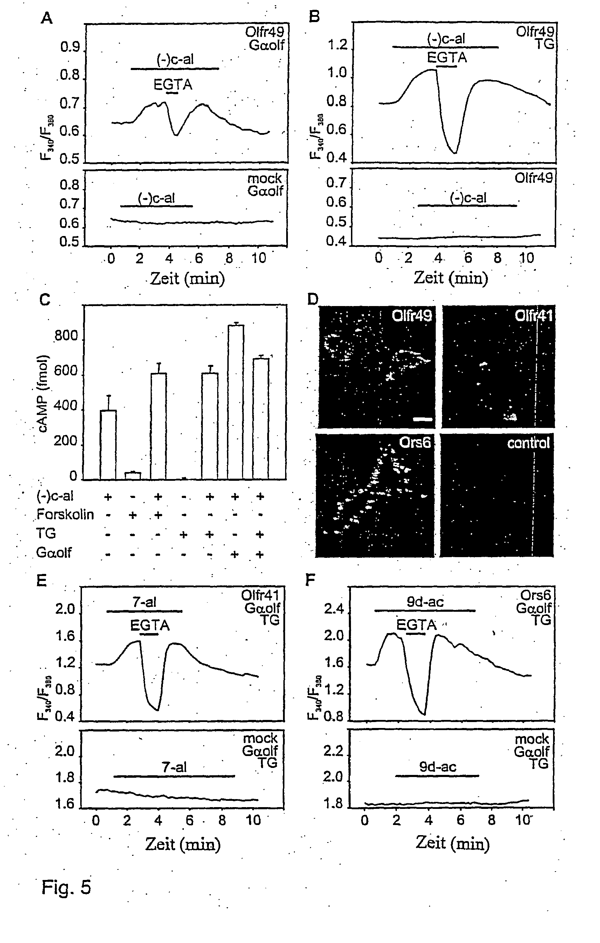

Olfaktorische Signaltransduktion und präferentielles Koppeln von OR via G-alpha-olf in He-La Zellen. In beiden Ca2+ Imaging-Experimenten und cAMP Assays mit HeLa-Cx43/CNGA2 Zellen zeigten die Erfinder, daß die Signaleffizienz von OR über den cAMP Signalweg durch die Vorbehandlung (Primen) mit entweder Forskolin oder Thapsigargin verbessert wird. Die Erfinder zeigten jedoch auch, daß die Anreicherung HeLa-Cx43/CNGA2 Zellen mit G Protein die Signaleffizienz von Rezeptoren erhöhte, so das der Bedarf einer Vorbehandlung mit Forskolin oder Thapsigargin dadurch beseitigt wurde. Daher scheint es wichtig zu sein, eine ausreichende Menge von Signal transduzierenden Komponenten zu exprimieren, die (-)Citronellal- oder Isoproterenol-induzierte cAMP Produktion wurde in HeLa-Cx43/CNGA2/rho-tag(39)-Olfr49 Zellen leicht beobachtet, sogar ohne eine Vorbehandlung mit Forskolin, Thapsigargin oder Überexpression von G-alpha-olf. Dies liegt wahrscheinlich an dem Koppeln von Rezeptoren über endogen exprimierte G-alpha-s. Die cAMP Tests zeigten weiter ein Koppeln von Olfr49 oder beta-AR an sowohl G-alpha-olf und G-alpha-s, und, zeigt darüber hinaus ein bevorzugtes Koppeln von Olfr49 oder beta-AR an jeweils G-alpha-olf oder G-alpha-s, wodurch frühere Ergebnisse bestätigt und erweitert werden (Jones et al., 1990; Kajiya et al., 2001; Liu et al., 2001). Der kleinere Unterschied in der OR-induzierten cAMP Produktion von G-alpha-olf gegenüber G-alpha-s angereicherten HeLa-Cx43/CNGA2/rho-tag(39)-Olfr49 Zellen, verglichen mit den Unterschieden in den beta-ARinduzierten cAMP Spiegeln (Fig. 4) kann durch eine schnellere Deaktivierung von G-alpha-olf-GTP durch die GTP Hydrolyse und die GTP Dissoziation relativ zu G-alpha-s-GTP (Liu et al., 2001) erklärt werden. Die Thapsigargin-induzierte Abnahme in der (-)Citronellal-stimulierten cAMP Produktion, die in HeLa-Cx43/CNGA2/rho-tag(39)-Olfr49 Zellen, die hG-alpha-olf exprimieren, beobachtet wurde (Fig. 5), kann durch eine Inhibierung von ACVI aufgrund einer erhöhten intrazelluären Ca2+ Konzentration erklärt werden.

Funktionelle Genomics mit einem OR Subgenom - Effekte der Odorant Konzentration Auf dem Niveau der OR-Ligand Interaktion ist ein kombinatorisches Kodieren allgemein akzeptiert, wie von oft breit "getunten" OSN abgeleitet. Die Annahme ist, daß ein OR durch verschiedene Odorantien aktiviert werden kann und daß ein Odorant mehrere OR mit verschiedenen Effizienzen aktivieren kann. Bis jetzt waren jedoch nur 14 OR Gensequenzen in einer (Malnic et al., 1999) jedoch nicht der anderen (Hamana et al., 2003) von zwei vergleichenden Untersuchungen publiziert, die versuchten, OR Sequenzen aus Hunderten von Einzelzell RT-PCR Experimenten mit Maus OSN die auf einen homologes Set von Odorantien regiert hatten, abzuleiten. In keiner dieser Untersuchungen wurden die abgeleiteten OR Sequenzen dazu verwendet, um die Odorant Responsivität ihrer Genprodukte zu bestätigen oder um ihre EC50-Rang Odorantienprofile mittels funktioneller heterologer Expression zu charakterisieren. Für einen der abgeleiteten putativen OR, Ors6 (Malnic et al., 1999), haben die Erfinder nun eine Odorant Responsivität in HeLa-Cx43/CNGA2/hG-alpha-olf Zellen gezeigt (Fig. 5), ähnlich zu der in OSN. In einer anderen Untersuchung wurde eine Bibliothek von rekombinanten OR Chimären in HEK-293 Zellen exprimiert, und funktionell mit Odorantien gescreened (Krautwurst et al., 1998). Aus diesem Experimenten hat sich Olfr49 als ein Responder für (-)Citronellal ergeben, den die Erfinder nun stabil exprimiert haben und durch sein EC50-Rang Odorantienprofil charakterisiert haben (Fig. 4).

In der vorliegenden Erfindung haben die Erfinder den Aspekt untersucht, daß das Odorant/Rezeptor Kodieren von der Konzentration des Odorant abhängt. Durch einen funktionellen Genomics Ansatz, wobei 93 Maus OR Zellinien gegenüber (-)Citronellal und (beta-Citronellol gescreent wurden, identifizierten die Erfinder 3 OR neu, die auf jeden Odorantien oder beide spezifisch antworten. Eine ansteigende Zahl von antwortenden OR (von 3-9% auf 22-59%) als eine Ergebnis einer ansteigenden Konzentration von (-)Citronellal läßt ein kombinatorisches Kodieren der Geruchsqualität und/oder -Intensität durch verschiedene OR Untergruppen vermuten. Dieses Phänomen wurde ursprünglich bei der Ca2+ Bildgebung oder bei elektrophysiologischen Experimenten bis jetzt nur mit OSN beobachtet, wobei 4 bis 57% der OSN auf ansteigende mikromolare Konzentrationen von bestimmten Odorantien antworteten (Sato et al., 1994; Malnic et al., 1999; Duchamp-Viret et al., 2000; Ma und Shepherd, 2000; Hamana et al., 2003). Die Zahl von OSN, und daher OR die verschiedene Odorantien erkannten, variierte in diesem Untersuchungen beträchtlich in Abhängigkeit von dem Odorantien und seiner Konzentration.

Die Erfinder beobachteten eine ähnliche Variation in ihren Experimenten, die möglicherweise an der Verwendung von verschiedenen OR Untergruppen liegt und teilweise auch experimentelle Variationen widerspiegeln kann, z.B. Unterschiede in der Transfektionseffizienz oder Unterschiede in der Plasmamembran-Expression. Ein Vorteil des unspezifischen gegenüber des spezifischen Tuning von OSN, und daher OR, auf die Qualität und die Intensitätskodierung mittels eines Gemisches von rezeptiven Feldern von größtmöglicher Diversifität wurde kürzlich in einem mathematischen Modell vorgeschlagen (Sanchez-Montanes und Pearce, 2002). Jedoch ist eine plötzliche Untersuchung vermuten, daß die prinzipiellen Odorqualitäten durch die am meisten sensitiven Rezeptoren für einen bestimmten Odoranten kodiert werden (Hamana et al., 2003).

Der menschliche OR1A1, der der orthologe OR (84%) zum Maus Olfr43 und LOC331758 ist, hat eine ähnliche Spezifität für (-)Citronellal beibehalten, wobei sein Konzentrations-Antwort-Verhältnis um den menschlichen Schwellenwert herum beginnt. Auf der anderen Seite können Olfr49 und viele andere OR als "Generalisten" für (-)Citronellal angesehen werden. Die Erfinder haben daher Beweise präsentiert, daß Olfr43 und LOC331758 in der Maus und OR1A1 und OR1A2 im Menschen Kandidaten dafür sind, Spezialisten für den OR für den Schlüssel Nahrungsmittel Odorantien (-)Citronellal zu sein. Auf lange Sicht kann jedoch nur ein holistischer Ansatz, zum Beispiel ein Screening aller menschlichen OR (Zozulya et al., 2001) und ein Etablieren von EC50-basierten Odorantienprofilen für alle antwortenden OR, zu einem vollständigen Bild darüber führen, welcher OR, oder welche Untergruppe von OR, die Odorqualität von z.B. Citronellal darstellt.

Die oben beschriebenen Ergebnisse wurden in Zusammenhang mit der Analyse eines bestimmten Odorantien ((-)Citronellal) und dessen entsprechenden Rezeptoren erhalten und diskutiert. Die beschriebenen Ergebnisse lassen sich jedoch ohne weiteres auch auf andere Rezeptor-Familien ausdehnen, ohne sich vom Bereich der vorliegenden Erfindung zu entfernen. Unter Heranziehung der hier beschriebenen Ergebnisse und der folgenden Beispiele kann der Fachmann die Verfahren der vorliegenden Erfindung leicht auf geeignete Weise anpassen, um so auch G Protein gekoppelte Rezeptoren anderer Klassen zu untersuchen und/oder aufzufinden. Das erfindungsgemäße Zellsystem ist somit innerhalb der G Protein gekoppelte Rezeptoren universell einsetzbar.

Die Erfindung soll nun im folgenden weiter durch die beigefügten Beispiele unter Bezug auf die beigefügten Figuren verdeutlicht werden, ohne jedoch darauf beschränkt zu werden.

In den Figuren zeigen:

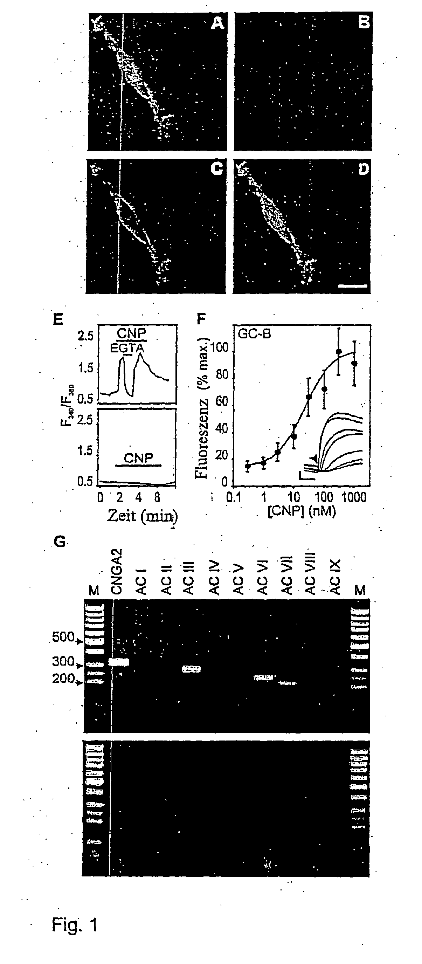

Figur 1. HeLa-Cx43/CNGA2 Zellen exprimieren den CNGA2 Kanal und die RNA für vier endogene Adenylylcyklasen. (A-D) Konfokale Fluoreszenzbilder von HeLa-Cx43/CNGA2 Zellen. (A) Permeabilisierte, anti-CNGA2/Alexa-488-markierte Zelle. (B) Primäre Antikörper ausgelassen. (C) Die Zelloberfläche wird mit Concanavalin A/Texas Red sichtbar gemacht. (D) Overlay von (A) und (C), mit ko-lokalisierten Signalen in Gelb. Skalenbalken, 20 µm. (E) CNP induzierte einen Ca2+ Influx in IBMX-vorbehandelte Zellen, transfiziert mit DNA für GC-B. Unteres Panel, Kontroll-transfizierte Zellen. Gemittelte Messungen von 6 Respondern/25 Gesamt-Zellen, und allen Zellen in der Kontrolle. (F) Konzentrations-Antwort Verhältnis von CNP. Die Daten sind Mittelwerte ± SD aus 5 Well Bestimmungen, EC50 = 0,027 µM. Einschub, Fluoreszenzmessungen. Pfeilspitze, Anwendung von CNP. Vertikale Skala, 200 Fluoreszenzzählungen; horizontale Skala, 1 min. (G) RT-PCR Produkte von HeLa-Cx43/CNGA2 mRNA, unter der Verwendung von Gen-spezifischen Primem für die olfaktorische CNGA2 Kanaluntereinheit und alle bekannten Typen von humanen AC (ACI-IX) (oberes Panel). Unteres Panel, -RT, wobei reverse Transkriptase ausgelassen wurde. M, Markergrößen (Basenpaare). Ähnliche Ergebnisse wurden in zwei unabhängigen Experimenten erhalten.

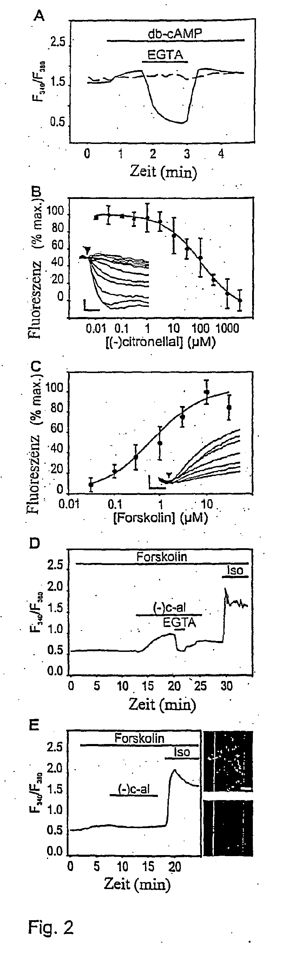

Figur 2: Tuning von CNGA2 für (-)Citronellal/Olfr49-induzierten Ca2+ Influx. (A) Aktivierung von CNGA2 durch db-cAMP in Thapsigargin- und IBMX-vorbehandelten HeLa-Cx43/CNGA2 Zellen, in Ca2+ bildgebenden Experimenten. Gestrichelte Linie, Zellen, denen CNGA2 fehlt. Gemittelte Messungen aus allen aufgezeichneten Zellen. (B) Konzentrations-Antwort Verhältnis von (-)Citronellal in einem FLIPR Experiment mit db-cAMPvorbehandelten Zellen, IC50 = 107,6 µM. Einschub, Fluoreszenzmessungen (0,003-3000 µM, von oben nach unten). Vertikale Skala, 200 Fluoreszenzzählungen; horizontale Skala, 30 Sek. (C) Konzentrations-Antwort Verhältnis von Forskolin Vorbehandlung (EC50 = 0,70 ± 0,40 µM) in Zellen, stimuliert mit (-)Citronellal (10 µM). Einschub, Fluoreszenzmessungen unter 0.03-30 µM Forskolin (von unten nach oben, max. Effekt = 10 µM). Vertikale Skala, 500 Fluoreszenzzählungen; horizontale Skala, 1 min. Daten in (B, C) sind Mittelwerte ± SD aus 5 Well Bestimmungen. Pfeilspitzen in Einschüben, Anwendung von (-)Citronellal. (D) (-)Citronellal(c-al)-induziertem Ca2+ Influx in Forskolin-vorbehandelte HeLa-Cx43/CNGA2/rho-tag(39)-Olfr49 Zellen. (E) Zellen, denen Olfr49 fehlt. Gemittelte Messungen aus allen aufgezeichneten Zellen. Horizontale Balken, Bad-Aufbringung von Wirkstoffen (10 µM). Iso, Isoproterenol. Ähnliche Ergebnisse wurden in zwei (A, B) oder drei (C, D) unabhängigen Experimenten erhalten. (E, rechtes oberes Panel) Konfokale Bilder von nicht-permeabilisierten B6-30/Alexa-488-markierten HeLa-Cx43/CNGA2/rho-tag(39)-Olfr49 Zellen. (E, rechtes unteres Panel), unter Auslassung der primären Antikörpers. Skalenbalken, 16 µm.

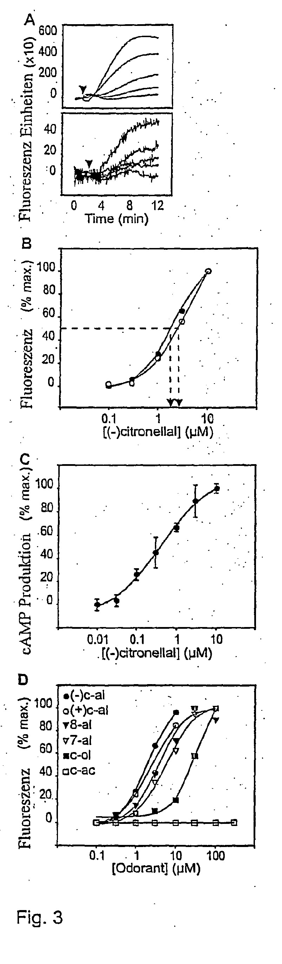

Figur 3: Odorant Spezifität und Konzentrationsbereiche von Olfr49. (A) (-)Citronellal-induzierter Ca2+ Influx in FLIPR Experimenten mit Forskolin-vorbehandelten Zellen. (A, oberes Panel) HeLa-Cx43/CNGA2/rho-tag(39)-Olfr49 Zellen; (A, unteres Panel) Beachte die zehnfach geringeren Amplituden in HeLa-Cx43/CNGA2 Zellen, die transient mit Olfr49 DNA transfiziert wurden. (B) Konzentrations-Antwort Verhältnisse mit den maximalen Amplituden abgeleitet von (A, oberes Panel, gefüllte Kreise) EC50 = 2,1 ± 0,07 µM, und (A, unteres Panel, offene Kreise) EC50 = 3,9 ± 1,3 µM. (C) (-)Citronellal-induzierte cAMP Produktion in HeLa-Cx43/CNGA2/rho-tag(39)-Olfr49 Zellen. Die Daten sind Mittel ± SD (n = 2 unabhängige Experimente), dargestellt als Prozent des Maximums (EC50 = 0,4 ± 0,25 µM). (D) Konzentrations-Antwort Verhältnisse von (-)Citronellal ((-)c-al, gefüllte Kreise), (+)Citronellal ((+)c-al, offene Kreise), Octanal (8-al, gefüllte Dreiecke), Heptanal (7-al, offene Dreiecke), beta-Citronellol (c-ol, gefüllte Quadrate) und Citronellinsäure (c-ac, offene Quadrate) auf Odorant-induzierten Ca2+-Influx in Forskolin-vorbehandelten HeLa-Cx43/CNGA2/rho-tag(39)-Olfr49 Zellen. Die Daten sind Mittelwerte von n = 3 unabhängigen Experimenten, mit SD < 20%.
Figur 4: Effekte von G-alpha-olf oder G-alpha-s Überexpression auf die Rezeptor/Ligandinduzierte cAMP Produktion. (A) RT-PCR unter der Verwendung von Gen-spezifischen Primem auf HeLa-Cx43/CNGA2 cDNA. - RT, reverse Transkriptase wurde ausgelassen. M, Markergrößen (Basenpaare). (B, C) HeLa-Cx43/CNGA2/rho-tag(39)-Olfr49 Zellen, transfiziert mit entweder G-alpha-olf oder G-alpha-s DNA (beides Ratte), IBMX-präinkubiert und stimuliert mit (-)Citronellal (B) oder Isoproterenol (C), beide bei 3 µM. Jeder Balken stellt das Mittel ± S. D. aus dreifachen Bestimmungen dar. Alle Unterschiede in der cAMP Produktion sind bei p<0.05 in (B) und (C) signifikant. Ähnliche Ergebnisse wurden in zwei unabhängigen Experimenten erhalten.
Figur 5: Effekte von Forskolin, Thapsigargin und G-alpha-olf auf Odorant/OR Signalgebung in HeLa-Cx43/CNGA2 Zellen. (A, E, F) Odorantien-induzierter Ca2+ Influx in Fura-2-beladene HeLa-Cx43/CNGA2/G-alpha-olf Zellen, transfiziert mit DNA für rho-tag(39)-Olfr49 (A), -Olfr41 (E), oder -Ors6 (F). Unteres Panel, Kontroll-transfizierte Zellen. (B) (-)Citronellal-induzierter Ca2+ Influx in Fura-2-beladene HeLa-Cx43/CNGA2/rho-tag(39)-Olfr49 Zellen ohne hG-alpha-olf. Die Konzentration der Odorantien ((-)c-al, (-)Citronellal; 8-al, Octanal; 9d-ac, Nonandioinsäure) war 10 µM. Beachte, daß die Zellen mit Forskolin, sondern mit Thapsigargin (TG) wo angegeben, behandelt wurden. Gezeigt sind mittlere Ca2+ Messungen von allen responsiven Zellen (8 Responder/30 gesamte Zellen bei (A), 4/25 bei (E), 12/21 bei (F), alle Zellen bei (B) und in der Kontrolle). Ähnliche Ergebnisse wurden in drei unabhängigen Experimenten erhalten. (C) cAMP Produktion in IBMX-vorbehandelten HeLa-Cx43/CNGA2/rho-tag(39)-Olfr49 Zellen, stimuliert mit (-)Citronellal (10 µM) in Anwesenheit von Forskolin (2,5 µM), Thapsigargin (1 µM) oder nach Transfektion mit G-alpha-olf (Ratte). Jeder Balken stellt das Mittel ± S. D. aus zwei unabhängigen Experimenten dar. (D) Konfokale Bilder von nicht-permeabilisierten B6-30/Alexa-488-markierten Zellen, die rho-tag(39)-OR exprimieren. Unteres rechtes Panel, Auslassen des primären Antikörpers. Skalenbalken, 16 µm.

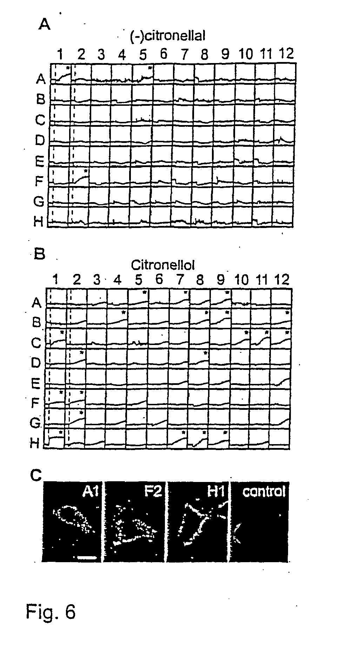

Figur 6: Screening von 93 OR Chimären mit (-)Citronellal und beta-Citronellol. (A, B) HeLa-Cx43/CNGA2/hG-alpha-olf Zellen transfiziert mit DNAs von 93 rho-tag(20)-M4 chimäre Maus OR und gegen 1 µM (A), oder 10 µM (B) von Odorant in FLIPR Experimenten gescreened. Gestrichelte Linien, Bad-Anwendung von Odorantien. Sternchen, Responder auf Odorantien. Die Koordinaten A10-A12 enthielten Kontroll-transfizierte Zellen. Beachte, daß das unterschiedliche Rauschsignal der einzelnen Messungen an der Normalisierung an die intrinsische Isoproterenol Signalamplitude in jedem Experiment liegt. Zeitskala, 4 min. (C) Konfokale Bilder von nicht-permeabilisierten und B6-30/Alexa-488-markierten Zellen, die rho-tag(20)-M4 OR Chimären exprimieren. Rechtes Panel, Auslassen des primären Antikörpers. Skalenbalken, 16 µm.

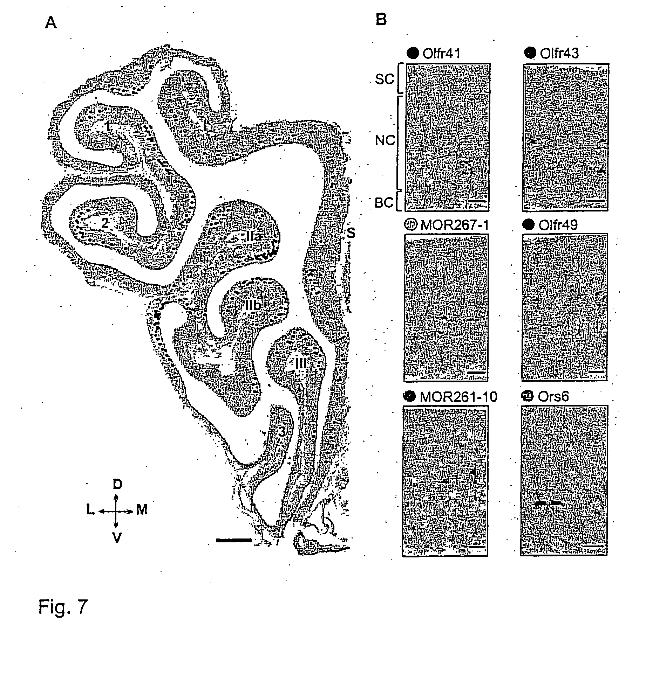

Figur 7: Differenzielle zonale Expressionsmuster von Maus OR *in situ* (A) *In situ* Hybridisierung von Odorantienrezeptor antisense RNA. Ein koronaler Halbschnitt von Maus OE mit Ectoturbinaten 1-3 und Endoturbinaten I-III faßt die Expressionsmuster von OR zusammen. Die Daten sind aus seriellen Schnitten, separat mit dem jeweiligen OR hybridisiert, wie durch gefärbte Punkte: Olfr41 (schwarz), Olfr43 (rot), MOR267-1 (gelb), Olfr49 (violett), 261-10 (grün), und Ors6 (blau) dargestellt. Skalenbalken, 300 µm. (B) Höhere Vergrößerung von einzelnen Schnitten von OE, hybridisiert mit antisense OR-Sonden. SC, Sustentakuläre Zellen; NC, neuronale Zellen; BC, Basalzellen. Skalenbalken, 10 µm.

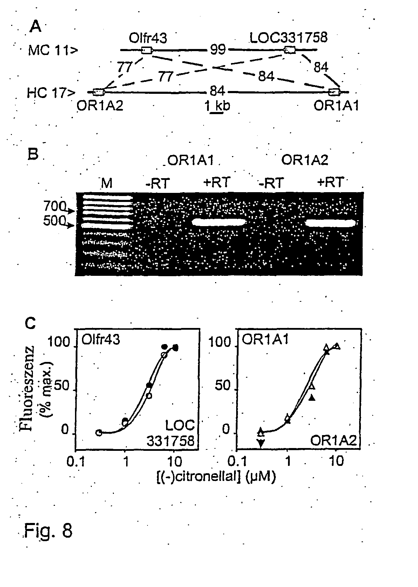

Figur 8: Genexpression und Funktion von humanen OR für (-)Citronellal - (A) Kandidaten-(-)Citronellal-Rezeptorgene in syntenischen Clustern auf Maus-Chromosom 11B3-B5 (MC 11) und humanem Chromosom 17p13.3 (HC 17). Die Pfeile zeigen den Bereich und die Orientierung der Gene an, auf Maßstab gezeichnet von NCBI Maus und humanen Genomkarten. Die Zahlen geben die Aminosäure-Identitäten (%) zwischen den Genprodukten an. (B) RT-PCR unter der Verwendung Gen-spezifischer Primer auf menschlicher olfaktorischer Epithel cDNA. -RT, reverse Transkriptase wurde ausgelassen. M, Markergrößen (Basepaare) (C) Konzentrations-Antwort Verhältnisse von (-)Citronellal für rho-tag(39)-Olfr43 (gefüllter Kreis), -LOC331758 (offener Kreis), -OR1A1 (gefülltes Dreieck), und -OR1A2 (offenes Dreieck) in HeLa-Cx43/CNGA2/G-alpha-olf Zellen. Ähnliche Ergebnisse wurden in drei unabhängigen FLIPR Experimenten erhalten. Pfeil, menschliche Schwellen-Konzentration (0,3 µM).

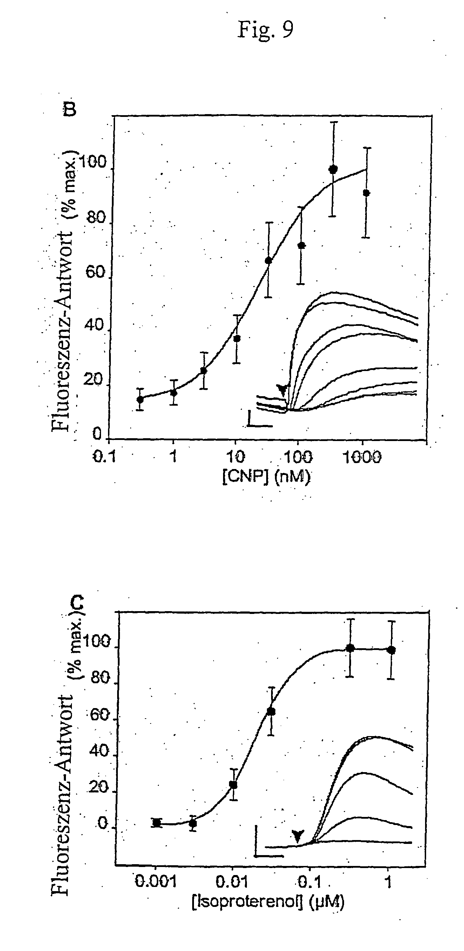

Figur 9: Schematische Darstellung des olfaktorischen Rezeptor-Signalwegs in HeLa-Zellen. Das System besteht aus dem Rezeptor (A), dem heterotrimeren G-Protein (B), der Adenylcyklase (C) und dem Kanal CNGA2 (D).

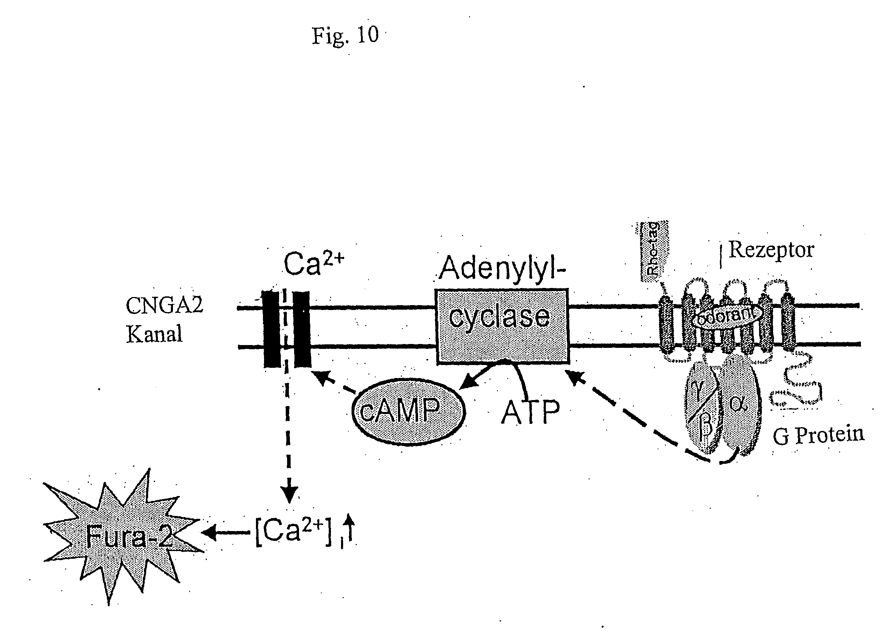

Figur 10: Generelle Geeignetheit des Zellsystems für die Charakterisierung von Rezeptoren, die die intrazelluläre Konzentration von zyklischen Nukleotiden modifizieren. (B) partikuläre Guanylylcyklase; (C) adrenerger Rezeptor.

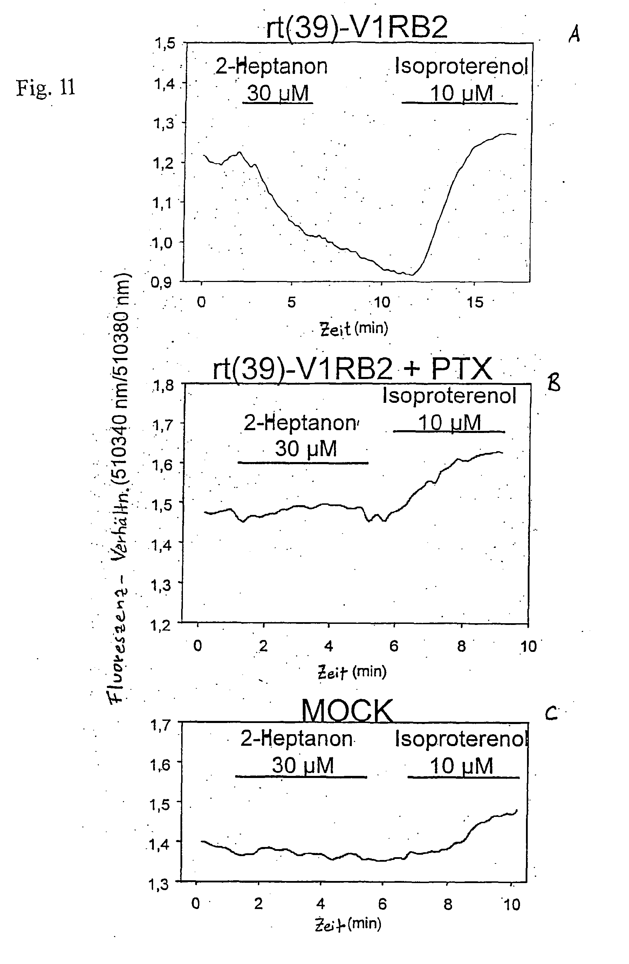

Figur 11: Generelle Geeignetheit des Zellsystems für die Charakterisierung von Rezeptoren, die die intrazelluläre Konzentration von zyklischen Nukleotiden modifizieren, am Beispiel eines Pheromonrezeptors rt(39)-V1R-b2 mit 2-Heptanon, (A). negative Kontrolle mit Pertussis-Toxin (B), das Toxin blockiert das spezifische G Protein G-alpha-I, (C) Leerkontrolle; alle drei Versuche mit Isoproterol, das auf die endogenen adrenergen Rezeptoren wirkt.
Figur 12: RT-PCR Produkte von a) HeLa/CNGA2 mRNA und b) HeLa/Olf Zellen unter der Verwendung von Gen-spezifischen Primern für die menschlichen Proteine Gαs und Gαolf. - RT = ohne reverse Transkriptase, M, Markergrößen (Basenpaare), c) Westernblot Analyse von HeLa/Cx43/CNGA2 Zellen (Gαs) und HeLa/Olf Zellen (Gαs und Gαolf). Der verwendete anti-αs-Antikörper erkennt beide G-Proteine Gαs und Gαolf mit einer Größe von 45 kDa. Die 41 kDa-Bande beruht auf Degradation oder ungenauer Expression. Die HeLa/Olf Zellen zeigen eine Überexpression der G-Proteine.

### Beispiele

Molekulare Klonierungen - Die Erfinder verwendeten Rhodopsin-markierten rho-Marker (20)-M4 chimäre Maus OR, jedoch eine andere Untergruppe von 93 OR, wie durch Krautwurst et al. (1998) berichtet. CDNA, die für den Rinder olfaktorische cyklisches Nukleotidöffnende Kanaluntereinheit CNGA2 (BTCAMPGC, X55010, Ludwig et al., 1990) kodierte, wurde in den Vektor pcDNA3.1/Zeo(+) eingefügt. Um die kodierende Region des olfaktorischen humanen Guaninnukleotid-bindenden Protein alpha (hG-alpha-olf, GNAL: NM002071) zu erhalten, isolierten die Erfinder zuerst humane RNA aus chirurgischen olfaktorischen Epithelbiopsien mit Trizol (Gibco), dann die mRNA mit Micro-FastTrack 2.0 (Invitrogen), und synthesierten erste-Strang cDNA unter der Verwendung von ImProm-II (Promega). Die Erfinder PCR-amplifizierten und subklonierten hG-alpha-olf in die CMV Promotor-angetriebene Expressionskassette pi2-dk, basierend auf Plasmid pIRES2-EGFP (Clontech), der jedoch der IRES-EGFP Teil fehlte. Die Erfinder PCR-amplifizierten die voll-Längen kodierenden Regionen von Maus Olfr49 (I-C6, NM010991) und Olfr41 (17, NM010983) (Krautwurst et al., 1998) von ihren Originalplasmiden, Maus Ors6 (NM020289, Malnic et al., 1999), Olfr43 (XM111129), LOC331758 (XM137710), MOR261-10 (NM146369), und MOR267-1 (NM146937) aus Maus (C57BL/6J) genomischer DNA, und die menschlichen OR1A1 (NM014565) und OR1A2 (NM012352) aus menschlicher genomischer DNA mit Pfu (Promega) oder PfuUltra (Stratagene). Die Amplikons wurden in pi2-dk(rt39) subkloniert.

Die Kassette stellt die ersten 39 Aminosäuren der Rinder-Rhodopsin (rho-tag(39)) als einen N-terminalen Marker zur Verfügung (Chandrashekar et al., 2000) für alle Vollängen OR. Die Identitäten aller subklonierter Amplikons wurde durch Sequenzierung überprüft (UKEHH, Hamburg).

Zellkultur und transiente DNA Transfektion - Alle Zellkulturmedien, Inhaltsstoffe und Antibiotika wurden von Invitrogen/Gibco erhalten, außer G418 (Calbiochem) und Puromycin (Sigma). HeLa Zellen wurden in Dulbecco's Modified Eagle Medium (DMEM) mit 10% hitze-inaktiviertem fötalem Rinderserum (FBS), 2 mM L-Glutamin, 100 U/ml Penicillin und 100 µg/ml Streptomycin in einer befeuchteten Atmosphäre (37°C, 5% CO2) angezogen.

Vor der Transfektion, wurden die Zellen auf Glas Deckgläser (VWR) für Einzelzell Ca2+ Bildgebung oder schwarze Wand/klarer Boden 96 Well Platten (Molecular Devices) für FLIPR, beide mit poly-D-Lysin beschichtet (10 µg/ml) ausgesät und bis zu einer präkonfluenten Monolayer angezogen. Die Zellen wurden mit DNA unter der Verwendung von Lipofektion (PolyFect, Quiagen) transfiziert, und 40 Stunden nach Transfektion in die Experimente aufgenommen.

Klonierung und Etablierung des Expressionsplasmids für humanes Galpha olf und der HeLa-Cx43/CNGA2/hG-alpha-olf (HeLa/Olf) Zellinie.
1) Um die kodierende Region des olfaktorischen menschlichen Guanin Nukleotid-bindenden G Proteins-alpha (hG-alpha-olf, GNAL: Accession-Number NM002071) zu erhalten, isolierten die Erfinder zuerst RNA aus menschlichen chirurgischen olfaktorischen Epithel-Biopsien mit Trizol (Gibco), wurde die dann mRNA mit Micro-FastTrack 2.0 (Invitrogen) isoliert und erste-Strang cDNA unter der Verwendung von ImProm-II (Promega) reverser Transkriptase (RT) synthetisiert. Die Erfinder PCR-amplifizierten die Vollängen kodierende Region von hG-alpha-olf unter der Verwendung einer Pfu DNA Polymerase (Promega,) und subklonierten diese in die CMV Promotor-angetriebene Expressionskassette pi2-dk, basierend auf Plasmid pIRES2-EGFP (Clontech), der jedoch die IRES-EGFP Teil fehlte.
2) Vor der Transfektion mit hG-alpha-olf, wurden HeLa-Cx43/CNGA2 Zellen in 100 mm Platten in einer Dichte von 1,6 x 10⁶ Zellen ausplattiert und über Nacht inkubiert. Die Zellen mit dem Expressionsplasmid hG-alpha-olf/pi2-dk, das die kodierende Regionen für hG-alpha-olf trug, durch Calciumphosphat Fällung transfiziert. HeLa/Olf Zellen wurden dann durch die Selektion von klonalen Populationen erhalten, die gegen 800 µg/ml G418 und responsiv gegenüber (-)Citronellal oder Isoproterenol waren. Diese wurden durch RT-PCR oder Westemblot bestätigt. Klonale Linien wurden in Standard DMEM, ergänzt mit Puromycin (1 µg/ml), Zeocin (100 µg/ml) und G418 (400 µg/ml) gehalten. Die Erfinder beobachteten eine stabile Expression von hG-alpha-olf bis mindestens Passage 10.

Einzelzell Ca2+ Bildgebung - Einzelzell Ca2+ Bildgebung wurde wie vorher beschrieben durchgeführt (Bufe et al., 2002). Die Fluoreszenz von einzelnen Fura-2-AM (Molecular Probes)-beladenen Zellen wurde bei einer Emissions-Wellenlänge von 515 nm nach Anregung mit 340 nm und 380 nm aufgezeichnet, und berechnet (F340/F380). Odoranten, natriuretisches Peptid Typ C (CNP), Forskolin, poly-D-Lysin, Probenecid, Salze, Puffers und db-cAMP (Dibutyryl cyklisches Adenosinmonophosphat, Membran-permeabel, verwendet bei 1 mM) waren von Sigma. IBMX (3-Isobutyl-1-methylxanthin), ein Blocker der Phosphodiesterase (100 µM, 30 min), war von Calbiochem. Die Forskolin Vorbehandlung (15 min) in Einzelzell Ca2+ Bildgebungs-Experimenten erfolgte immer bei 10 µM, wurde jedoch in allen Experimenten mit HeLa-Cx43/CNGA2/hG-alpha-olf Zellen weggelassen. Thapsigargin (Bio-Trend/Tocris; 1 µM, 30 min), ein Blocker einer intrazellulären Ca2+-ATPase, wurde verwendet, um cytoplasmatische Spiegel von Ca2+ zu erhöhen, was daher die OR-aktivierte cAMP Signalgebung via Ca2+-sensitver ACIII (Choi et al., 1992) erleichterte, oder um jede Rezeptor-vermittelte Ca2+Freisetzung von IP3-sensitiven internen Lagerstätten zu verhindern (Thastrup et al., 1994). Bad-Anwendug von 1 mM EGTA (Sigma) wurde verwendet um einen Influx von extrazellulärem Ca2+ in die Zellen zu bestätigen. Alle Chemikalien waren von der höchsten erhältlichen Reinheit.

Fluoreszenz Imaging Platten Lesegerät (FLIPR) Assay - Das FLIPR (Molecular Devices) integriert eine Argonlaser Anregungsquelle, einen 96 Well Pipettor, und ein Nachweissystem auf dem System, das eine CCD (Charged Coupled Device) Bildkamera einschließt. Die Experimente wurden mit FLUO-4 (4 µM, Molecular Probes)-beladenen Zellen durchgeführt. Eine Vorbehandlung mit Forskolin (2,5 µM, 15 min) oder IBMX (100 µM, 30 min) erfolgte vor der Anwendung der Agonisten. Die Agonist-Antwortamplituden wurden aus der Peakstimulierten Fluoreszenz der Lösungmittel Kontrolle-substrahiert und Basislinien-korrigierten Messungen bestimmt, und über 4-5 Wells gemittelt, die den selben Rezeptor exprimierten und den selben Reiz erhielten.

Datenanalyse - In den FLIPR Screening Experimenten wurden Responder wurden nach dem Kriterium von ≥50% von der maximalen Fluoreszenzzunahme innerhalb der ersten Minute nach Odorant Anwendung ausgewählt. Odorant-induzierte Fluoreszenzamplituden wurden an die durch 10 µM Isoproterenol in demselben Well hervorgerufene Amplitude normalisiert, am Ende des Experiments. Die EC50 oder IC50 Werte und Kurven wurden aus Anpassen der Funktion f(x) = (a-d)/(1+(x/C)^nh)+d an die Daten durch nicht-lineare Regression, mit a = Minimum, d = Maximum, C = EC50 oder IC50, und *n*H = Hill Koeffizient abgeleitet.

Immunocytochemie - Die Plasmamembranexpression von CNGA2 oder rho-tag-OR wurde unter der Verwendung der primären Antikörper Kaninchen-anti-CNG2 (Alomone) oder B6-30 (Margrave et al., 1986) ermittelt, die gegen den C-Terminus von CNGA2 oder den N-terminalen Teil von Rhodopsin gerichtet waren, in jeweils permeabilisierten oder nicht-permeabilisierten Zellen. Die Zelloberfläche wurde durch Nachweisen von Plasmamembran Glycoprotein mit 20 µl/ml Biotin-konjugiertem Concanavalin A (Sigma) und Färbung mit Avidin-konjugiertem Texas Red (Molecular Probes) sichtbar gemacht, wie früher beschrieben (Bufe et al., 2002). Markierte CNGA2 oder OR Proteine wurden unter der Verwendung von Alexa-488-gekoppelten sekundären Antikörpern (Ziege-anti -Kaninchen, -anti- Maus, Molecular Probes) und konfokaler Mikroskopie (Leica TCS SP2 Laser Scan) sichtbar gemacht.

cAMP Test - HeLa-Cx43/CNGA2/rho-tag(39)-Olfr49 Zellen (10⁶ Zellen pro Well in 6-Well Platten) wurden nicht-transfiziert verwendet, oder wurden mit 1,5 µg Ratten G-alpha-olf oder Ratten G-alpha-s DNA transfiziert, unter der Verwendung von PolyFect. Nach 40 Stunden wurden die Zellen mit IBMX (100 µM, 30 min) präinkubiert und gegenüber (-)Citronellal oder Isoproterenol (2 min) ausgesetzt. Die Prästimulation mit Forskolin wurde allgemein ausgelassen. Die cAMP Spiegel wurden mit dem ¹²⁵J-markierten cAMP Testsystem (Amersham) in dreifachen Bestimmungen gemessen. Die Werte wurden vor der Agonist-Behandlung gegen den Spiegel von cAMP normalisiert. Um die durchschnittliche cAMP Konzentration/Zelle abzuschätzen, ermittelten die Erfinder das durchschnittliche Sphärenvolumen durch Messen der Durchmesser von 125 runden Zellen-Morphen unter der Verwendung eines Axioplan Mikroskops (Zeiss) und der Metamorph Software (Universal Imaging Corp.). Die µmol cAMP, produziert bei der jeweiligen Odorantien EC50 wurden von nicht-linearer Regressionsanalyse erhalten.

Gewebe und Schnittpräparation und *in situ* Hybridisierung - OE wurde von männlichen erwachsenen C57BL/6J Mäusen erhalten. Sechs Wochen alte betäubte Mäuse wurden transkardial mit eiskaltem PBS perfundiert und mit Bouin's Lösung (Sigma) fixiert. Die *in situ* Hybridisierung wurde bei 65°C mit den jeweiligen Digoxigenin-markierten sense und antisense Ribosonden, hergestellt mit dem DIG RNA Markierungs-Mix (Roche) auf seriellen koronalen 14 µm Kryoschnitten, durchgeführt.

Stabile Rekonstitution von Odorantien/OR-induzierter cAMP/Ca2+ Influx Signalübertragung in HeLa Zellen - Anfänglich etablierten die Erfinder die humane HeLa-Cx43/CNGA2 Zellinie, die stabil den olfaktorischen homomeren CNGA2 Kanal als ein Reporter exprimierte, um Rezeptor-induzierten Erhöhungen in intrazellulärem cyklischen Nukleotiden online zu verfolgen. Die Erfinder bestätigten die Expression von mRNA für den CNGA2 Kanal in HeLa-Cx43/CNGA2 Zellen durch RT-PCR, und die Plasmamembran-Expression des CNGA2 Proteins durch Immunocytochemie (Fig. 1). Der homomere CNGA2 Kanal weist eine EC50 für cGMP (3 µM) auf, die 20-fach niedriger als die für cAMP (Finn et al., 1998). Die cGMP Signalgebung kann in vivo durch eine Untergruppe von OSN verwendet werden, einschließlich der olfaktorischen partikulären Guanylylcyklase GC-D (Fulle et al., 1995). Die Erfinder testeten die Möglichkeit, eine Erhöhung in cGMP zu verfolgen, durch Transfizieren der Ratten partikulären Guanylylcyklase Typ B (GC-B) in HeLa-Cx43/CNGA2 Zellen, für die das C Typ natriuretische Peptid (CNP) der bekannte Agonist ist (Lucas et al., 2000). In diesen Zellen rief CNP in der Anwesenheit von IBMX einen GC-B-abhängigen und EGTA-sensitiven Ca2+ Influx hervor, mit einem EC50 von 0,027 µM (± 0,001 SD; n =2 ) (Fig. 1). Eine Charakterisierung von HeLa-Cx43/CNGA2 Zellen durch RT-PCR ergab die Expression von mRNA für G-alpha-s (Fig. 4) sowie für ACIII, VI, VII, und IX (Fig. 1). In diesen Zellen, in der Anwesenheit von Forskolin und ansteigenden Konzentrationen des P-adrenergen Rezeptor-(P-AR)-Agonisten Isoproterenol, riefen die Erfinder die cAMP Produktion und Ca2+ Influx mit EC50 Werten von jeweils 0,028 ± 0,004 µM und 0,026 ± 0,008 µM, hervor. Die Erfmder schlossen daraus, daß in diesen Zellen die OR/Odorantien-induzierte cAMP Signalübertragung und der Ca2+ Influx durch die CNGA2 Kanäle etabliert werden kann. Jedoch ließ die Arbeit von Kurahashi und Mitarbeitern vermuten, daß Odorantien selber auch die Antwort von OSN unterdrücken können, durch direktes Blockieren ihrer CNG oder Spannungs-öffnenden Kanäle (Kurahashi et al., 1994; Kawai, 1999). Daher testeten die Erfinder zuerst ansteigende Konzentrationen von mehreren Citronellin Odorantien und aliphatischen Aldehyden auf inhibitorische Effekte auf den CNGA2-abhängigen Ca2+ Influx in HeLa-Cx43/CNGA2 Zellen, der direkt durch db-cAMP aktiviert wurde (Fig. 2). (-)Citronellal, das eines der -400 Schlüssel Nahrungsmittel Odorantien ist (Grosch, 2001), blockierte den Ca2+ Influx in diese Zellen bei Konzentrationen von höher als 3 µM, und mit einer IC50 von 131,7 ± 33,9 µM (n = 2) (Fig. 2). Octanal und Heptanal bis zu 100 µM, beta-Citronellol bis zu 3 mM, und Citronellinsäure bis zu 300 µM blockierten den db-cAMP-induzierten Ca2+ Influx durch CNGA2 nicht (n = 2). Um die OR/Odorant-induzierte cAMP Signalübertragung und den Ca2+ Influx in HeLa- Cx43/CNGA2 Zellen zu untersuchen, etablierten die Erfinder die stabile Expression von rho-tag(39)-Olfr49 für den (-)Citronellal als der kognate Odorant identifiziert wurde (Krautwurst et al., 1998). Die Erfinder bestätigten seine Expression auf dem Plasmamembran Niveau durch Immunocytochemie und konfokale Mikroscopie (Fig. 2). Das Diterpen Forskolin (Seamon und Daly, 1986) kann dazu verwendet werden, um direkt Typ I-VIII der 9 Säuger AC (für eine Zusammenfassung siehe Smit und Iyengar, 1998) zu aktivieren. In den Händen der Erfinder mußten HeLa-Cx43/CNGA2 Zellen mit Forskolin präinkubiert werden, um einen Rezeptor-induzierten und CNGA2-abhängigen Ca2+ Influx zu messen. Dies liegt möglicherweise an einer suboptimalen Expression von G-alpha-s, da in Ca2+ bildgebenden Experimenten beide Überexpressionen von G-alpha-s (n = 3) und G-alpha-olf (Fig. 5) die Prästimulation von AC durch Forskolin kompensieren konnten. Die Erfinder bestimmten eine prä-stimulierende Konzentration von 10 µM Forskolin, um einen anschließenden maximalen Odorantien-induzierten Ca2+ Influx in HeLa-Cx43/CNGA2/rho-tag(39)-Olfr49 Zellen zu erlauben (Fig. 2). In diesen Zellen stimulierte, wenn mit 10 µM Forskolin vorbehandelt, (-)Citronellal bei 10uM einen OR-abhängigen Ca2+ Influx der vollständig durch extrazelluläres EGTA antagonisiert werden konnte (Fig. 2). Der quantitative Vergleich der relativen Potenzen der Agonisten durch die Bestimmung ihrer EC50 Werte ist ein Standardverfahren der GPCR und Agonisten-Klassifizierung. Die Erfinder verwendeten daher das FLIPR System, um Konzentrations-Antwortkurven für kognate Rezeptor-Ligandenpaare innerhalb von Konzentrationsbereichen zu etablieren, wo keine oder eine geringe Blockade von CNGA2-abhängigem Ca2+ Influx beobachtet wurde. FLIPR Experimente mit HeLa-Cx43/CNGA2 Zellen, die stabil rho-tag(39)-Olfr49 exprimierten, ergab einen EC50 Wert für (-)Citronellal von 2,1 ± 0,3 µM (n = 3), verglichen zu 4,0 ± 2,2 µM (n = 5) in Zellen mit transient transfiziertem rho-tag(39)-Olfr49 (Fig. 3). In der rho-tag(39)-Olfr49 stabilen Zellinie induzierte (-)Citronellal eine cAMP Produktion auf eine Konzentrations-abhängige Weise (Fig. 3), mit einem EC50 Wert von 0,49 µM (± 0,25 SD; n = 2). Um eine Korrelation zwischen der Agonist-induzierten cAMP Produktion, Ca2+ Influx und Aktivierung des CNGA2 Kanals zu stärken, berechneten die Erfinder eine durchschnittliche cAMP Konzentration von 48 µM oder 68 µM innerhalb einer einzelnen HeLa- Cx43/CNGA2 Zelle bei den EC50 Konzentrationen für jeweils (-)Citronellal oder Isoproterenol. Diese Agonist/GPCR-induzierte durchschnittliche intrazelluläre cAMP Konzentration ließ sich gut mit der EC50 von 65 µM für cAMP auf dem homomeren olfaktorischen CNGA2 Kanal vergleichen, die in electrophysiologischen Experimente mit inside-out Patches von CNGA2-exprimierenden HEK293 Zellen (Finn et al., 1998) erhalten wurde. Durch Testen von aliphatischen Aldehyd- und Citronellal-verwandten Verbindungen auf HeLa-Cx43/CNGA2/rho-tag(39)-Olfr49 Zellen erhielten die Erfinder das Odorantienprofil: (-)Citronellal > (+)Citronellal > Octanal > Heptanal >> beta-Citronellol, widergespiegelt durch die EC50 Werte von jeweils 2,1 ± 0,1 µM (n = 4), 2,6 ± 0,4 µM (n = 3), 3,7 ± 0,1 µM (n = 3), 6,0 ± 1,0 µM (n = 3) und 32,8 ± 2,7 µM (n = 3) (Fig. 3).

Stabile Rekonstitution von Odorantien/OR-induzierter cAMP Signalübertragung via G-alpha-olf - HeLa-Cx43/CNGA2 Zellen exprimieren die mRNA für die G Protein Untereinheit alpha-s, jedoch nicht für G-alpha-olf (Fig. 4). Um die Effizienz von beiden G Proteinen bei der OR Signalübertragung zu untersuchen, transfizierten die Erfinder die DNA für rG-alpha-olf oder rG-alpha-s in HeLa-Cx43/CNGA2/rho-tag(39)-Olfr49 Zellen, und maßen die (-)Citronellal- und via endogenem beta-AR, Isoproterenol-induzierte cAMP Produktion. Bei Fehlen von Forskolin oder Thapsigargin, erhöhte die Transfektion mit G-alpha-olf oder G-alpha-s bevorzugt die Signalübertragungseffizienz von jeweils rho-tag(39)-Olfr49 oder endogenem beta-AR (Fig. 4). Um die OR Signalübertragung zu verbessern, etablierten die Erfinder daher die stabile Expression von humanem G-alpha-olf in HeLa-Cx43/CNGA2 Zellen. (-)Citronellal rief einen Ca2+ Influx in HeLa-Cx43/CNGA2/hGaolf Zellen hervor, die rho-tag(39)-Olfr49 exprimierten (Fig. 5) und mit einer ähnlichen EC50 (2,2 µM), wie in Forskolin-vorbehandelten HeLa-Cx43/CNGA2/rho-tag(39)-Olfr49 Zellen (vergl. Fig. 3). Unter den vier AC die in den vorliegenden HeLa-Cx43/CNGA2 Zellen exprimiert werden (s. Fig. 1), kann nur die olfaktorische ACIII durch Ca2+, in der Anwesenheit eines aktiven G Proteins alpha Untereinheit (für eine Zusammenfassung, siehe Smit und Iyengar, 1998) aktiviert werden. Die Erfinder stellten daher die Hypothese auf, daß eine Zunahme in der intrazellulären Ca2+ Konzentration durch ein Vorbehandeln der Zellen mit Thapsigargin, die cAMP Signalübertragung von OR spezifisch via ACIII erleichtern wird. Die Erfinder beobachteten an Odorantien-induzierten Ca2+ Influx in HeLa-Cx43/CNGA2/rho-tag(39)-Olfr49 Zellen ohne hG-alpha-olf nur dann, wenn die Zellen mit Thapsigargin vorbehandelt wurden (Fig. 5). In HeLa-Cx43/CNGA2/hGaolf Zellen, die rho-tag(39)-Olfr49 exprimierten und mit Thapsigargin vorbehandelt wurden, rief (-)Citronellal einen ähnlichen Ca2+ Influx wie in nicht-vorbehandelten Zellen (Fig. 5) hervor. Die Erfinder testeten dann die drei Parameter 'Forskolin Vorbehandlung', 'Thapsigargin Vorbehandlung', und 'hG-alpha-olf Anreicherung' in cAMP Tests mit (-)Citronellal-stimulierten HeLa-Cx43/CNGA2/rho-tag(39)-Olfr49 Zellen. Die (-)Citronellal-induzierte cAMP Produktion wurde signifikant erhöht und zu dem selben Ausmaß in Zellen, die mit entweder Forskolin oder Thapsigargin vorbehandelt wurden, verglichen zu einer Stimulation mit (-)Citronellal in Zellen ohne Vorbehandlung (Fig. 5). Die Transfektion mit hG-alpha-olf führte zu der höchsten (-)Citronellal-induzierten cAMP Produktion. In diesen Zellen reduzierte Thapsigargin die (-)Citronellal-induzierte cAMP Produktion auf einen ähnlichen Spiegel, wie in Thapsigargin- oder Forskolin-vorbehandelte Zellen ohne hG-alpha-olf (Fig. 5) gemessen wurde. Um die Validität des erfindungsgemäßen Zellsystems für OR Signalübertragung zu testen, verwendeten die Erfinder zwei andere OR, Olfr41 und Ors6, für die die kognaten Odorantien Heptanal und Nonandionsäure jeweils in Ca2+ bildgebenden Experimenten mit rekombinanten OR, oder isolierten ORN identifiziert wurden (Krautwurst et al., 1998; Malnic et al., 1999). In den Händen der Erfinder löste Heptanal bei 10 µM den Ca2+ Influx in Thapsigargin vorbehandelte HeLa- Cx43/CNGA2/hG-alpha-olf Zellen aus, die rho-tag(39)-Olfr41 exprimierten (Fig. 5). In Thapsigargin-vorbehandelten HeLa-Cx43/CNGA2/hG-alpha-olf Zellen, die rho-tag(39)-Ors6 exprimierten, induzierte Nonandionsäure (Fig. 5), jedoch keine andere C8 Carbon- oder Dicarbonsäure oder C9 Carbonsäure (n = 2) eine Ca2+ Influx bei 10 µM. Ähnliche Ergebnisse wurden mit rho-tag(39)-Olfr41 und rho-tag(39)-Ors6 in HeLa-Cx43/CNGA2/hG-alpha-olf Zellen ohne Thapsigargin-Vorbehandlung erhalten (n = 2).

Screening und funktionelle Identifikation von Maus OR für Citronellin Odorantien - Durch Analyse des "Human Genome Projects" fanden die Erfinder keine gut definierten Orthologe (-85% Aminosäure-Identität) für Olfr49. Die am engsten verwandten OR in der Maus oder dem Menschen zeigen jeweils 54% oder 56% Aminosäure-Identität zu Olfr49. Jedoch wurden Sensitivitäts-abhängige hierarchische Rezeptorcodes für Gerüche in a kürzlichen Untersuchung vorgeschlagen (Hamana et al., 2003). Die Erfinder fingen daher an, andere OR Kognate für (-)Citronellal mit höherer Effizienz und Spezifität als Olfr49 zu identifizieren. Um den kombinatorischen Aufwand des Screening von Hunderten von Odorantien versus -1000 OR zu vermeiden, screenten Erfinder ungefähr 10% eines gesamten Maus OR Genoms, exprimiert in HeLa-Cx43/CNGA2/hG-alpha-olf Zellen. Die Erfinder testeten zuerst beide Odorantien bei Konzentrationen unterhalb ihrer EC50 Werte für Olfr49, und (-)Citronellal nahe ihrer Geruchsschwelle im Menschen (0,3 µM, Leffingwell, 2003). In FLIPR Experimenten, wendeten die Erfinder 1 µM (-)Citronellal oder 10 µM beta-Citronellol auf HeLa-Cx43/CNGA2/hG-alpha-olf Zellinien an, die 93 aus einer Sammlung von 141 rho-tag(20)-M4 chimären Maus OR exprimierten. Die 93 hier getesteten OR sind exklusiv der 80 OR, die in einer vorhergehenden Studie getestet wurden (Krautwurst et al., 1998). Jedoch wurde die rho-tag(20)-M4 Chimäre von Olfr49 als eine positive Kontrolle eingeschlossen (96-Well Koordinate A5) und antwortete entsprechend auf beide Odorantien (Fig. 6). Die Erfinder identifizierten weiter zwei OR Chimären (3%, 96 Well Koordinaten A1 und F2) die zu 1 µM (-)Citronellal antworteten, wobei F2 auch auf 10 µM beta-Citronellol (Fig. 6) reagierte. 20 OR Chimären (22%) einschließlich rho-tag(20)-M4-Olfr49 (A5), antworteten auf 10 µM beta-Citronellol (Fig. 6), wobei H1 die stärkste normalisierte Antwort zeigte. Ein Erhöhen der Konzentration von (-)Citronellal von 1 µM auf 3 µM erhöhte die Zahl von antwortenden OR Chimären auf 40 (43%), einschließlich der OR Chimären an den Koordinaten A1, A5 und F2. Die Erfinder beobachteten einen ähnlichen Anstieg im Prozentsatz von antwortenden OR in drei anderen Screening-Experimenten mit einer Untergruppe von 67 OR Chimären, die teilweise mit den hier gezeigten 93 OR überlappten, wenn die Konzentration von (-)Citronellal von 1 µM (9%, n = 1) auf 3 µM (22% und 59%) erhöht wurde. Die OR Chimären A5 und F2 wurden in diesen Experimenten eingeschlossen und antworteten in allen Fällen auf beide Konzentration von (-)Citronellal. Keine der 93 OR Chimären antwortete auf Citronellinsäure bis zu 10 µM (n = 2). Blasting der TMII-VII Sequenzen der drei neu identifizierten Responder aus den 96 Well Koordinaten A1, F2 und H1 gegen die GenBank ergab die entsprechenden Maus OR Gene, jeweils Olfr43, mOR267-1, und mOR261-10. Diese OR zeigen 35-41% Aminosäure-Identität untereinander und mit Olfr49. Die A1, F2 und H1 rho-tag(20)-M4-Chimären sowie ihre entsprechenden rho-tag(39)-Vollängen OR antworteten selektiv auf 1 µM (-)Citronellal (Olfr43, Olfr49, mOR267-1) oder 10 µM beta-Citronellol (Olfr49, mOR267-1, mOR261-10) in Einzelzell Ca2+ bildgebenden Experimenten, wenn in Thapsigargin-vorbehandelten HeLa-Cx43/CNGA2/hG-alpha-olf Zellen (n = 3) exprimiert.

Spatiale Genexpression in den OE von OR für Citronellin Odorantien - Der Olfr43 unterscheidet sich bis jetzt von allen anderen OR, die von den Erfindern als responsiv gegenüber Citronellin Odoranten identifiziert wurden. Olfr43 zeigte eine Spezifität für (-)Citronellal bei 1 µM über 10 µM von anderen Citronellin Odorantien, wie zum Beispeil beta-Citronellol (siehe Fig. 7) oder Citronellinsäure (n = 2). Die Erfinder stellten daher die Hypothese auf, daß sich die spezifische Funktion von Olfr43 in einer topographischen Expression in den OE widerspiegelt, die sich von der Expression der anderen OR unterscheidet. In *in situ* RNA Hybridisierungs-Experimenten untersuchten die Erfinder die Genexpression von Olfr41, Olfr43, Olfr49, MOR261-10, MOR267-1, und Ors6 in OSN der Maus OE. Ors6 und Olfr41 die auf die aliphatischen Odorantien Nonandionsäure und Heptanal antworteten, markierten jeweils die am meisten dorso-medialen und ventro-lateralen Expressionsmuster in koronalen Schnitten von Maus OE (Fig. 7). Dazwischen zeigten die (-)Citronellal und beta-Citronellol Responder Olfr49, MOR261-10 und MOR267-1 eine überlappende zonale Expression, mit einer lateral verschobenen Expression von Olfr43 (Fig. 7). Die funktionelle Identifikation eines menschlichen orthologen OR für Maus Olfr43. Eine Charakterisierung von syntenischen OR Cluster des menschlichen Chromosom 17p13.3 und Maus Chromosom 11B3-B5 führte zu der Identifikation von orthologen OR (Glusman et al., 2000; Lapidot et al., 2001). OR1A1 und OR1A2 wurden als die zwei engsten menschlichen Homologe to Olfr43 gefunden, die jeweils 84% und 77% Aminosäure-Identität mit Olfr43 und ihrem engsten Maus-Homolog, LOC331758 (99%) teilen (Fig. 8, Lapidot et al., 2001). RT-PCR Experimente zeigten eine mRNA Expression von OR1A1 und OR1A2 in menschlichem olfaktorischem Epithel (Fig. 8). Wenn in HeLa-Cx43/CNGA2/hG-alpha-olf Zellen exprimiert, zeigten rho-tag(39)-Olfr43, - LOC331758, -OR1A1, und -OR1A2 ähnliche EC50 Werte für (-)Citronellal, jeweils 2,1 ± 0,2 µM (n = 3), 3,2 ± 0,8 µM (n = 3), 2,2 ± 0,4 µM (n = 3), und 2,4 ± 0,7 µM (n = 2), (Fig. 8).

### Referenzen:

Araneda R. C., Kini A. D. und Firestein S. (2000) The molecular receptive range of an odorant receptor. NatNeurosci,3,1248-55.
Bakalyar H. A. und Reed R. R. (1990) Identification of a specialized adenylyl cyclase that may mediate odorant detection. Science 250, 1403-6.
Baker H., Cummings D. M., Munger S. D., Margolis J. W., Franzen L., Reed R. R. und Margolis F. L. (1999) Targeted deletion of a cyclic nucleotide-gated channel subunit (OCNC1): biochemical und morphological consequences in adult mice. J Neurosci,19,9313-21.
Belluscio L, Gold G. H., Nemes A. und Axel R. (1998) Mice deficient in G(olf) are anosmic. Neuron,20,69-81.
Bozza J., Feinstein P., Zheng C. und Mombaerts P. (2002) Odorant receptor expression defines functional units in the mouse olfaktorisch system. J Neurosci,22,3033-43.
Brunet L. J., Gold G. H. und Ngai J. (1996) General anosmia caused by a targeted disruption of the mouse olfactory cyclic nucleotide-gated cation channel. Neuron 7,681 -93.
Buck L. und Axel R. (1991) A novel multigene family may encode odorant receptors: a molecular basis for odor recognition. Cell,65,175-87.
Bufe B., Hofmann T., Krautwurst D., Raguse J. D. und Meyerhof W. (2002) The human TAS2R16 receptor mediates bitter taste in response to beta-glucopyranosides. Nat Genet 32,397-40).
Chandrashekar J., Mueller K. L, Hoon M. A., Adler E., Feng L, Guo W., Zuker C. S. und Ryba N. J. (2000) T2Rs function as bitter taste receptors. Cell, 100,703-11.
Chess A., Simon I., Cedar H. und Axel R. (1994) Allelic inactivation regulates olfactory receptor gene expression. Cell,78,823-34.
Choi E. J., Xia Z. und Storm D. R. (1992) Stimulation of the type III olfaktorisch adenylyl cyclase by calcium und calmodulin. Biochemistry,31, 6492-8.
Crider J. Y. und Sharif N. A. (2002) Adenylyl cyclase activity mediated by beta- adrenoceptors in immortalized human trabecular meshwork und non-pigmented ciliary epithelial Zellen. J Ocul Pharmacol Ther,18,221-30.
Dhallan R. S., Yau K. W., Schrader K. A. und Reed R. R. (1990) Primary structure and functional expression of a cyclic nucleotide-activated channel from olfactory neurons. Nature, 347,184-7.
Duchamp-Viret P., Duchamp A. und Chaput M. A. (2000) Peripheral odor coding in th rat and frog: quality and intensity specification. J Neurosci,20,2383-90.
Dzeja C., Hagen V., Kaupp U. B. und Frings S. (1999) Ca2+ permeation in cyclic nucleotide-gated channels. Embo J,18,131-44.
Finn J. T., Krautwurst D., Schroeder J.E., Chen J. Y., Reed R. R. und Yau K. W. (1998) Functional co-assembly among subunits of cyclic-nucleotide-activated, nonselective cation channels, and across species from nematode to human. Biophys J, 74,1333-45.
Firestein S., Darrow B. und Shepherd G. M. (1991) Activation of the sensory current in salamander olfactory receptor neurons depends on a G protein-mediated cAMP second messenger system. Neuron, 6,825-35.
Fulle,H.J., Vassar,R., Foster.D.C., Yang,R.B., Axel,R. und Garbers,D.L. (1995) A receptor guanylyl cyclase expressed specifically in olfactory sensory neurons. Proc Natl Acad Sci U S A92,3571 -5.
Gaillard I., Rouquier S., Pin J. P., Mollard P., Richard S., Barnabe C., Demaille J. und Giorgi D. (2002) A single olfactory receptor specifically binds a set of odorant molecules. Eur J Neurosci,15,409-18.
Glusman G., Sosinsky A, Ben-Asher E., Avidan N., Sonkin D., Bahar A., Rosenthal A., Clifton S., Roe B., Ferraz C., Demaille J. und Lancet D. (2000) Sequence, structure, and evolution of a complete human olfactory receptor gene cluster. Genomics,63,227-45.
Gold G. H. (1999) Controversial issues in vertebrate olfactory transduction. Annu Rev Physiol,61,857-71.
Grosch W. (2001) Evaluation of the key odorants of foods by dilution experiments,aroma models and omission. Chem Senses,26,533-45.
Hamana H., Hirono J., Kizumi M. und Sato T. (2003) Sensitivity-dependent Hierarchical Receptor Codes for Odors. Chem Senses.28,87-104.
Hargrave P. A., Adamus G., Arendt A., McDowell J. H., Wang J., Szaby A., Curtis D. und Jackson R. W. (1986) Rhodopsin's amino terminus is a principal antigenic site. Exp Eye Res.42,363-73.
Jones D. T., Masters S. B., Bourne H. R. und Reed R. R. (1990) Biochemical characterization of three stimulatory GTP-binding proteins. The large and small forms of Gs und the olfaktorisch-specific G-protein.Golf. J Biol Chem,265,2671-6.
Jones D. T. und Reed R. R. (1989) Golf: an olfactory neuron specific-G protein involved in odorant signal transduction. Science,244,790-5.
Juilfs D. M., Fulle H. J., Zhao A. Z., Houslay M. D., Garbers D. L. und Beavo J. A. (1997) A subset of olfaktorisch neurons that selectively express cGMP-stimulated phosphodiesterase (PDE2) and guanylyl cyclase-D define a unique olfactory signal transduction pathway. Proc Natl Acad Sci U S A94,3388-95.
Kajiya K., Inaki K., Tanaka M., Haga J., Kataoka H. und Touhara K. (2001) Molecular bases of odor discrimination: Reconstitution of olfactory receptors that recognize overlapping sets of odorants. J Neurosci,21,6018-25.
Kawai F. (1999) Odorants suppress T- und L-type Ca2+ currents in olfactory receptor celles by shifting their inactivation curves to a negative voltage [In Process Citation]. Neurosci Res,35,253-63.
Krautwurst D., Yau K. W. und Reed R. R. (1998) Identification of ligands für olfactory receptors by functional expression of a receptor library. Cell,95,917-26.
Kurahashi J., Lowe G. und Gold G. H. (1994) Suppression of odorant responses by odorants in olfactory receptor cells. Science,265,118-20.
Lapidot X., Pilpel Y., Gilad Y., Falcovitz A., Sharon D., Haaf J. und Lancet D. (2001) Mouse-human orthology relationships in an olfactory receptor gene cluster. Genomics,71,296-306.
Leffingwell J. C. (2003) Chirality & Odour Perception - Acyclic Terpenoid Odorants. http://www.leffingwell.com/chirality/acyclic_terpenoid.htm.
Liu H. Y., Wenzel-Seifert K. und Seifert R. (2001) The olfaktorisch G protein G(alphaolf) possesses a lower GDP-affinity and deactivates more rapidly than G(salphashort): consequences for receptor-coupling and adenylyl cyclase activation. J Neurochem,78, 325-38.
Lucas K. A., Pitari G. M., Kazerounian S., Ruiz-Stewart I., Park J., Schulz S., Chepenik K. P. und Waldman S. A. (2000) Guanylyl cyclases and signaling by cyclic GMP. Pharmacol Rev,52,375-414.
Ludwig J., Margalit J., Eismann E., Lancet D. und Kaupp U. B. (1990) Primary structure of cAMP-gated channel from bovine olfactory epithelium. FEBS Lett,270,24-9.
Malvl. und Shepherd G. M. (2000) Functional mosaic organization of mouse olfactory receptor neurons. Proc Natl Acad Sci USA,97,12869-74.
Malnic B., Hirono J., Sato T. und Buck L. B. (1999) Combinatorial receptor codes für odors. Cell,96,713-23.
Meyer M. R., Angele A., Kremmer E., Kaupp U. B. und Muller F. (2000) A cGMP-signaling pathway in a subset of olfactory sensory neurons. Proc Natl Acad Sci USA,97,10595-600.
Mombaerts P. (1999) Molecular biology of odorant receptors in vertebrates. Annu Rev Neurosci,22,487-509.
Nakamura T. und Gold G. H. (1987) A cyclic nucleotide-gated conductance in olfactory receptor cilia. Nature,325,442-4.
Omura M., Sekine H., Shimizu J., Kataoka H. und Touhara K. (2003) In situ Ca2+ imaging of odor responses in a coronal olfactory epithelium slice. Neuroreport.14,1123-7.
Reed R. R. (1992) Signaling pathways in odorant detection. Neuron,8,205-9.
Sanchez-Montanes M. A. und Pearce T. C. (2002) Why do olfactory neurons have unspecific receptive fields? Biosystems,67,229-38.
Sato T., Hirono J., Tonoike M. und Takebayashi M. (1994) Tuning specificities to aliphatic odorants in mouse olfactory receptor neurons and their local distribution. J Neurophysiol, 72,2980-9.
Scott J.W. und Brierley T. (1999) A functional map in rat olfactory epithelium. Chem Senses,24,679-90.
Scott J. W., Brierley T. und Schmidt F. H. (2000) Chemical determinants of the rat electroolfactogram. J Neurosci,20,4721-31.
Seamon K. B. und Daly J. W. (1986) Forskolin: its biological and chemical properties. Adv Cyclic Nucleotide Protein Phosphorylation Res,20,1-150.
Sicard G. und Holley A. (1984) Receptor cell responses to odorants: similarities und differences among odorants. Brain Res,292,283-96.
Smit M J. und lyengar R. (1998) Mammalian adenylyl cyclases. Adv Second Messenger Phosphoprotein Res, 32,1-21.
Thastrup O., Dawson A. P., Scharff O., Foder B., Cullen P. J., Drobak B. K., Bjerrum P. J., Christensen S. B. und Hanley M. R. (1994) Thapsigargin, a novel molecular probe für studying intracellular calcium release and storage. 1989. Agents Actions,43, 187-93.
Touhara K. (2002) Odor discrimination by G protein-coupled olfactory receptors. Microsc Res Tech,58,135-41.
Touhara K., Sengoku S., Inaki K., Tsuboi A., Hirono J., Sato T., Sakano H. und Haga T. (1999) Functional identification and reconstitution of an odorant receptor in single olfactory neurons. Proc Natl Acad Sci USA, 96, 4040-5.
Wetzel C. H., Oles M., Wellerdieck C., Kuczkowiak M., Gisselmann G. und Hatt H. (1999) Specificity and sensitivity of a human olfactory receptor functionally expressed in human embryonic kidney 293 Zellen und Xenopus Laevis oocytes. J Neurosci 19,7426-33.
Wong S. T., Trinh K., Hacker B., Chan G. C., Lowe G., Gaggar A., Xia Z., Gold G. H. und Storm D. R. (2000) Disruption of the type III adenylyl cyclase gene leads to peripheral and behavioral anosmia in transgenic mice. Neuron, 27,487-97.
Young J. M. und Trask B. J. (2002) The sense of smell: Genomics of vertebrate odorant receptors. Hum Mol Genet, 11,1153-60.
Zhang X. und Firestein S. (2002) The olfactory receptor gene superfamily of the mouse. Nat Neurosci,5,124-33.
Zhao H., Ivic L., Otaki J. M., Hashimoto M., Mikoshiba K. und Firestein S. (1998) Functional expression of a mammalian odorant receptor. Science,279,237-42.
Zozulya S., Echeverri F. und Nguyen J. (2001) The human olfactory receptor repertoire. Genome Biol,2,RESEARCH0018.

## Patentansprüche

1. Rekombinantes Zellsystem, umfassend eine tierische Wirtszelle, umfassend die folgenden rekombinanten Proteine
- einen rekombinanten spezifischen G Protein-gekoppelten Rezeptor,
- den rekombinanten Ca2+ permeabler Kanal CNGA2, und
- ein rekombinantes Protein aus der Gruppe der Connexine, z. B. Cx43 oder Cx26.

2. Rekombinantes Zellsystem nach Anspruch 1, weiterhin umfassend eine auf den spezifischen G Protein-gekoppelten Rezeptor abgestimmte Cyklase, z. B. eine Adenylyl- oder Guanylyl-Cyklasen.

3. Rekombinantes Zellsystem nach Anspruch 1 oder 2, wobei der rekombinante spezifische G Protein-gekoppelte Rezeptor ausgewählt ist aus der Gruppe der Pheromonrezeptoren, z.B. des V1R-Typs mit allen Familien VR-a bis VR-1, einschließlich der V3R-Typs (VR-d), zum Beispiel V1R-b2, der Hormonrezeptoren, z. B. der beta-adrenergen Rezeptoren und der olfaktorischen Rezeptoren, z. B. OR1A1, OR1A2, Olfr43, Olfr49, MOR261-10, MOR267-1, LOC331758, Olfr41 oder Olfr6.

4. Rekombinantes Zellsystem nach Anspruch 1, 2, oder 3, weiterhin umfassend ein auf den spezifischen G Protein-gekoppelten Rezeptor abgestimmtes rekombinantes G-Protein, z. B. G-alpha-olf.

5. Rekombinantes Zellsystem nach einem der vorgenannten Ansprüche, wobei die tierische Wirtszelle ausgewählt ist aus Mauszellinien oder humanen Zellinien, z.B. menschlichen Krebszellinien, wie z. B. HeLa oder HEK293.

6. Rekombinantes Zellsystem nach einem der vorgenannten Ansprüche, wobei das Zellsystem einen potentiellen rekombinanten spezifischen G Protein-gekoppelten Rezeptor umfaßt.

7. Rekombinantes Zellsystem nach Anspruch 5, ausgewählt aus den Zellsystemen HeLa-Cx43/CNGA2/Olfr49; HeLa-Cx43/CNGA2/G-alpha-olf; HeLa-Cx43/CNGA2/G-alpha-olf/Olfr 49; HeLa-Cx43/CNGA2/G-alpha-olf/Olfr41; HeLa-Cx43/CNGA2/G-alpha-olf/Olfr 6 oder HeLa-Cx43/CNGA2/G-alpha-olf/OR1A1.

8. Rekombinantes Zellsystem nach einem der vorgenannten Ansprüche, wobei die rekombinanten Proteine stabil und/oder transient transfiziert vorliegen.

9. Rekombinantes Zellsystem HeLa-Cx43/CNGA2/G-alpha-olf, wie am 20. April 2004 bei der DSMZ - Deutsche Sammlung von Mikroorganismen und Zellkulturen GmbH im Mascheroder Weg 1b, D-38124 Braunschweig unter der Hinterlegungsnummer DSM ACC2649 hinterlegt.

10. Verfahren zur Herstellung eines rekombinanten Zellsystems, umfassend - zur Verfügung stellen einer tierischen Wirtszelle,
- Einbringen eines rekombinanten spezifischen G Protein-gekoppelten Rezeptors oder eines potentiellen rekombinanten spezifischen G Protein-gekoppelten Rezeptors,
- Einbringen des rekombinanten Ca2+ permeablen Kanals CNGA2, und
- Einbringen eines rekombinanten Proteins aus der Gruppe der Connexine, z. B. Cx43 oder Cx26.

11. Verfahren nach Anspruch 10 -, weiterhin umfassend - Einbringen einer auf den spezifischen G Protein-gekoppelten Rezeptor abgestimmte Cyklase, z. B. eine Adenylyl- oder Guanylyl-Cyklase.

12. Verfahren nach einem der Ansprüche 10 oder 11, weiterhin umfassend - Einbringen eines auf den spezifischen G Protein-gekoppelten Rezeptor abgestimmten rekombinanten G-Proteins, z. B. G-alpha-olf.

13. Verfahren nach einem der Ansprüche 10 bis 12 -, wobei das Einbringen ausgewählt ist aus (Ca2+-Phosphat-)Transfektion, Lipofektion oder Elektroporation, sowie anschließender optionaler Integration ins Genom mit Hilfe einer Rekombinase und/oder Antibiotika-Selektionsklonierung, und Transduktion.

14. Verfahren zur Auffindung von Rezeptor aktivierenden Substanzen, umfassend die Schritte von
- zur Verfügung stellen eines rekombinanten Zellsystems nach einem der Ansprüche 1 bis 5 oder 7 bis 9,
- in Kontakt bringen des Zellsystems mit einer potentiell G Protein-gekoppelten Rezeptor aktivierenden Substanz, und
- Messen der Aktivierung oder Inhibierung des Ca2+ Einstroms in die Zelle.

15. Verfahren nach Anspruch 14, wobei die potentiell G Protein-gekoppelte Rezeptor induzierende Substanz ausgewählt ist aus Odorantien, wie zum Beispiel (-)Citronellal oder beta-Citronellol, Pheromonen, Hormone, wie zum Beispiel Adrenalin oder natriuretisches Peptid Typ-C.

16. Verfahren nach Anspruch 14 oder 15, wobei das Messen des Ca2+ Einstroms in die Zelle eine Beladung der Zelle mit Fura-2-AM oder Fluo-4-AM und ein Messen der Emissions-Wellenlänge bei 515 nm einschließt.

17. Verfahren nach einem der Ansprüche 14 bis 16, wobei das Zellsystem mit einem Enhancer, wie zum Beispiel Forskolin oder Thapsigargin vorbehandelt wird.

18. Verfahren zur Herstellung einer pharmazeutischen Zusammensetzung, umfassend die Schritte von
- Durchführen eines Verfahrens nach einem der Ansprüche 14 bis 17, und
- Formulieren der erhaltenen G Protein-gekoppelten Rezeptor induzierenden Substanz mit bekannten Hilfs- und Zusatzstoffen.

19. Verfahren zur Auffindung von G Protein-gekoppelten Rezeptoren, umfassend die Schritte von
- zur Verfügung stellen eines rekombinanten Zellsystems nach Anspruch 6,
- in Kontakt bringen des Zellsystems mit einer Rezeptor-aktivierenden Substanz oder vermutlich Rezeptor-aktivierenden Substanz, und
- Messen der Aktivierung oder Inhibierung des Ca2+ Einstroms in die Zelle.

20. Verfahren nach einem der Ansprüche 14 bis 19, wobei das Verfahren in einer Hochdurchsatz-Umgebung erfolgt, z.B. in Mikrotiterplatten in einem Fluoreszenz-Plattenlesegerät oder hochauflösend mikroskopgestützt auf Einzelzellebene.

21. Verwendung eines rekombinanten Zellsystems nach einem der Ansprüche 1 bis 5 und 7 bis 9 zur Deorphanisierung von zellulären G Protein-gekoppelten Rezeptoren durch Auffindung von Rezeptor induzierenden Substanzen, z.B. Odorantien.

22. Verwendung eines rekombinanten Zellsystems nach Anspruch 6 zur Auffindung von zellulären G Protein-gekoppelten Rezeptoren.

## Claims

1. Recombinant cell system, comprising an animal host cell comprising the following recombinant proteins
- a recombinant specific G protein-coupled receptor,
- the recombinant Ca2+ permeable channel CNGA2, and
- a recombinant protein from the group of connexines, e.g. Cx43 or Cx26.

2. Recombinant cell system according to claim 1, further comprising a cyclase that is adapted to the specific G protein coupled receptor, e.g. an adenylyl or a guanylyl cyclase.

3. Recombinant cell system according to claim 1 or 2, wherein the recombinants specific G protein coupled receptor is chosen from the group of pheromone receptors, e.g. of the V1R-type with all families VR-a to VR-1, including the V3R-type (VR-d) for example V1R-b2, of the hormone receptors, for example the beta-adrenergic receptor and of the olfactory receptors, e.g. OR1A1, OR1A2, Olfr43, Olfr49, MOR261-10, MOR267-1, LOC331758, Olfr41 or Olfr6.

4. Recombinant cell system according to claims 1, 2, or 3, further comprising a recombinant G protein adapted to the specific G protein coupled receptor, e.g. G-alpha-olf.

5. Recombinant cell system according to one of the previous claims, wherein the animal host cell is selected from mouse cell lines or human cell lines, e.g. human cancer cell lines, such as HeLa or HEK293.

6. Recombinant cell system according to one of the previous claims, wherein the cell system comprises a potential recombinant specific G protein coupled receptor.

7. Recombinant cell system according to claim 5, chosen from the cell systems HeLa-Cx43/CNGA2/Olfr49; HeLaCx43/CNGA2/G-alpha-olf; HeLa-Cx43/CNGA2/G-alpha-olf/Olfr 49; HeLa-Cx43/CNGA2/G-alpha-olf/Olfr41; HeLaCx43/CNGA2/G-alpha-olf/Olfr6 or HeLa-Cx42/CNGA2/G-alpha-olf/OR1A1.

8. Recombinant cell system according to one of the previous claims, wherein the recombinant proteins are present stably and / or in a transiently transfected manner.

9. Recombinant cell system HeLa-Cx43/CNGA2/G-alpha-olf, as deposited on April 20, 2004 with the DSMZ (Deutsche Sammlung von Mikroorganismen und Zellkulturen GmbH) Mascheroder Weg 1b, D-38124 Braunschweig under the deposit no. DSM ACC2649.

10. Method for producing a recombinant cell system, comprising
- providing an animal host cell,
- introducing a recombinant specific G protein coupled receptor or a potential recombinant specific G protein coupled receptor,
- introducing the recombinant Ca2+ permeable channel CNGA2, and
- introducing a recombinant protein from the group of the connexines, e.g. Cx43 or Cx26.

11. Method according to claim 10, further comprising - introducing a cyclase that is adapted to the specific G protein coupled receptor, e.g. a adenylyl or guanylyl cyclase.

12. Method according to one of claims 10 or 11, further comprising - introducing a recombinant G protein that is adapted to the specific G protein coupled receptor, e.g. G-alpha-olf.

13. Method according to claims 10 to 12, wherein the introducing is chosen from (Ca2+ phosphate) transfection, lipofection or elecroporation, as well as subsequent optional integration into the genome based on a recombinase and / or antibiotic selection cloning, and transduction.

14. Method for finding receptor activating substances, comprising the steps of
- providing a recombinant cell system according to one of claims 1 to 5 or 7 to 9,
- contacting the cell system with a potential G protein coupled receptor activating substance, and
- measuring the activation or inhibition of the Ca2+ influx into the cell.

15. Method according to claim 14, wherein the potential G protein receptor inducing substance is chosen from odorants, as for example (-)citronellal or beta-cintronellol, pheromons, hormones, as for example adrenaline or natriuretic peptide type-C.

16. Method according to claim 14 or 15, wherein the measuring of the Ca2+ influx into the cell includes charging the cell with Fura-2-AM or Fluo-4-AM and measuring the emission wave length at 515 nm.

17. Method according to one of claims 14 to 16, wherein the cell system has previously been treated with an enhancer, such as for example forskoline or thapsigargin.

18. Method for producing a pharmaceutical composition, comprising the steps of
- performing a method according to one of claims 14 to 17, and
- formulating the obtained G protein coupled receptor inducing substance with known excipients and additives.

19. Method for finding G protein coupled receptors, comprising the steps of
- providing a recombinant cell system according to claim 6,
- contacting the cell system with a receptor activating substance or presumably a receptor activating substance, and
- measuring the activation or inhibition of the Ca2+ influx into the cell.

20. Method according to one of claims 14 to 19, wherein the method is performed in a high throughput manner, e.g. in microtiter plates in a fluorescence plate-reading device or in high resolution microscope-based on a single-cell level.

21. Use of the recombinant cell system according to one of claims 1 to 5 and 7 to 9 for deorphanising of cellular G protein coupled receptors through finding receptor inducing substances, e.g. odorants.

22. Use of a recombinant cell system according to claim 6 for finding of cellular G protein coupled receptors.

## Revendications

1. Système cellulaire recombinant, comprenant une cellule hôte animale, comprenant les protéines recombinantes suivantes
- un récepteur couplé à une protéine G spécifique recombinant,
- le canal CNGA2 recombinant perméable au Ca2+, et
- une protéine recombinante provenant du groupe des connexines, par exemple, Cx43 ou Cx26.

2. Système cellulaire recombinant selon la revendication 1, comprenant en outre une cyclase adaptée au récepteur couplé à une protéine G spécifique, par exemple, une adénylyl cyclase ou des guanylyl cyclases.

3. Système cellulaire recombinant selon la revendication 1 ou 2, dans lequel le récepteur couplé à une protéine G spécifique recombinante est sélectionné dans le groupe des récepteurs de phéromones, par exemple du type V1R avec toutes les familles VR-a à VR-1, y compris du type V3R (VR-d), par exemple V1R-b2, des récepteurs d'hormones, par exemple, des récepteurs bêta-adrénergiques et des récepteurs olfactifs, par exemple, OR1A1, OR1A2, Olfr43, Olfr49, MOR261-10, MOR267-1, LOC331758, Olfr4l ou Olfr6.

4. Système cellulaire recombinant selon la revendication 1, 2 ou 3, comprenant en outre une protéine G recombinante adaptée au récepteur couplé à une protéine G spécifique, par exemple, G-alpha-olf.

5. Système cellulaire recombinant selon l'une quelconque des revendications précédentes, dans lequel la cellule hôte animale est sélectionnée parmi des lignées cellulaires de souris ou des lignées cellulaires humaines, par exemple des lignées cellulaires cancéreuses humaines, comme par exemple HeLa ou HEK293.

6. Système cellulaire recombinant selon l'une quelconque des revendications précédentes, dans lequel le système cellulaire comprend un récepteur couplé à une protéine G spécifique recombinant potentiel.

7. Système cellulaire recombinant selon la revendication 5, sélectionné parmi les systèmes cellulaires HeLa-Cx43/CNGA2/Olfr49 ; HeLa-Cx43/CNGA2/G-alpha-olf; HeLa-Cx43/CNGA2/G-alpha-olf/Olfr 49 ; HeLa-Cx43/CNGA2/G-alpha-olf/Olfr41 ; HeLa-Cx43/CNGA2/G-alpha-olf/Olfr 6 ou HeLa-Cx43/CNGA2/G-alpha-olf/OR1A1.

8. Système cellulaire recombinant selon l'une quelconque des revendications précédentes, dans lequel les protéines recombinantes sont présentes sous forme stable et/ou transfectée de manière transitoire.

9. Système cellulaire recombinant HeLa-Cx43/CNGA2/G-alpha-olf tel que déposé le 20 avril 2004 auprès de la DSMZ (Deutsche Sammlung von Mikroorganismen und Zellkulturen GmbH), sise à Mascheroder Weg 1b, 38124 Brunswick, Allemagne, sous le numéro de dépôt DSM ACC2649.

10. Procédé de préparation d'un système cellulaire recombinant, comprenant
- la mise à disposition d'une cellule hôte animale,
- l'introduction d'un récepteur couplé à une protéine G spécifique recombinant ou d'un récepteur couplé à une protéine G spécifique recombinant potentiel,
- l'introduction du canal CNGA2 recombinant perméable à Ca2+, et
- l'introduction d'une protéine recombinante provenant du groupe des connexines, par exemple Cx43 ou Cx26.

11. Procédé selon la revendication 10, comprenant en outre
- l'introduction d'une cyclase adaptée au récepteur couplé à une protéine G spécifique, par exemple une adénylyl cyclase ou guanylyl cyclase.

12. Procédé selon l'une quelconque des revendications 10 ou 11, comprenant en outre
- l'introduction d'une protéine G recombinante adaptée au récepteur couplé à une protéine G spécifique, par exemple, G-alpha-olf.

13. Procédé selon l'une quelconque des revendications 10 à 12, dans lequel l'introduction est sélectionnée parmi la transfection (de phosphate de Ca2+), la lipofection ou l'électroporation, ainsi que l'intégration optionnelle subséquente dans le génome à l'aide d'une recombinase et/ou d'un clonage sélectif aux antibiotiques, et la transduction.

14. Procédé pour la détection de substances activant un récepteur, comprenant les étapes consistant à
- mettre à disposition un système cellulaire recombinant selon l'une quelconque des revendications 1 à 5 ou 7 à 9,
- mettre en contact le système cellulaire avec une substance activant éventuellement un récepteur couplé à une protéine G, et
- mesurer l'activation ou l'inhibition de l'afflux de Ca2+ dans la cellule.

15. Procédé selon la revendication 14, dans lequel la substance induisant éventuellement un récepteur couplé à une protéine G est sélectionnée parmi des substances odorantes comme, par exemple, le (-)citronellal ou le bêta-citronellol, des phéromones, des hormones comme, par exemple, l'adrénaline ou le peptide natriurétique de type C.

16. Procédé selon la revendication 14 ou 15, dans lequel la mesure de l'afflux de Ca2+ dans la cellule inclut un chargement de la cellule en fura-2-AM ou fluo-4-AM et une mesure de la longueur d'onde d'émission à 515 nm.

17. Procédé selon l'une quelconque des revendications 14 à 16, dans lequel le système cellulaire est traité au préalable avec un amplificateur comme, par exemple, la forskoline ou la thapsigargine.

18. Procédé de préparation d'une composition pharmaceutique, comprenant les étapes consistant à
- réaliser un procédé selon l'une quelconque des revendications 14 à 17, et
- formuler, avec des auxiliaires et des additifs connus, la substance induisant un récepteur couplé à une protéine G obtenue.

19. Procédé pour la détection de récepteurs couplés à une protéine G, comprenant les étapes consistant à
- mettre à disposition un système cellulaire recombinant selon la revendication 6,
- mettre en contact le système cellulaire avec une substance activant un récepteur ou une substance activant probablement un récepteur, et
- mesurer l'activation ou l'inhibition de l'afflux de Ca2+ dans la cellule.

20. Procédé selon l'une quelconque des revendications 14 à 19, dans lequel le procédé s'effectue dans un environnement à haut débit, par exemple dans des plaques de microtitration dans un lecteur de plaques de fluorescence ou à haute résolution à l'aide d'un microscope au plan de la cellule individuelle.

21. Utilisation d'un système cellulaire recombinant selon l'une quelconque des revendications 1 à 5 et 7 à 9 pour la désorphanisation de récepteurs cellulaires couplés à une protéine G par la détection de substances induisant un récepteur, par exemple de substances odorantes.

22. Utilisation d'un système cellulaire recombinant selon la revendication 6 pour la détection de récepteurs cellulaires couplés à une protéine G.
